# EUROPEAN PATENT SPECIFICATION

(11) **EP 1 525 309 B1**
(45) Date of publication and mention of the grant of the patent: **18.04.2012**
(21) Application number: 03740832.5
(22) Date of filing: 18.07.2003
(51) Int. Cl.: C12N 15/10

(54) **STABILISATION OF NUCLEIC ACIDS**
STABILISIERUNG VON NUKLEINSÄUREN
STABILISATION D'ACIDES NUCLEIQUES

(30) Priority: 02.08.2002 GB 0217963
(43) Date of publication of application: 27.04.2005
(73) Proprietor: Cyclops Genome Sciences Limited, Cambridge CB1 2AL (GB)
(72) Inventor: GOLDSBOROUGH, Andrew, Simon, F-34980 St Gely du Fesc (FR)
(74) Representative: Daniels, Jeffrey Nicholas
(86) International application number: PCT/GB2003/003131
(87) International publication number: WO 2004/013155

(56) References cited:
- WO-A-00/75302
- WO-A-01/94626
- US-A- 5 804 683
- J.H.BOAL ET AL.: "Cleavage of Oligodeoxyribonucleotides from controlled-pore glass supports and their rapid deprotection." NUCLEIC ACIDS RESEARCH., vol. 15, 1996, pages 3115-3117, XP002267799 OXFORD UNIVERSITY PRESS, SURREY., GB ISSN: 0305-1048 cited in the application

## Description

### Field of the Invention

The present invention relates to a method for the stabilisation and partial or complete isolation of nucleic acids including DNA and RNA.

### Background to the Invention

Various methods are known for the purification of nucleic acids such as (i) the use of a salt chaotrope and silica surfaces, (ii) a phenol based extraction, and (iii) a chaotrope and precipitation of the nucleic acid. Methods for the purification of nucleic acids have been extensively described (Sambrook et al., (1989) Molecular Cloning: A Laboratory Manual, CSH).

WO 00/75302 discloses a method of isolating RNA.

WO 01/94626 discloses a method of producing a double-stranded oligo - or poly-nucleotide from a template.

These methods do not provide a method to stabilise the analyte nucleic acid prior to its extraction, rather the nucleic aicds are extracted as quickly as possible from the sample in order to minimise degradation. Other drawbacks of these methods are that the purified nucleic acids are contaminated with proteins and other biomolecules that lead to inhibition of downstream enzymatic applications and possible cleavage of the nucleic acid. Many of the methods are also time consuming and result in loss of a significant proportion of the desired nucleic acid analyte. Indeed one of the downsides of obtaining higher purity nucleic acid is that the overall yield of the nucleic acid is reduced

### Summary of the Invention

The present invention aims to overcome disadvantages in the prior art.

Accordingly, in a first aspect, the present invention provides a method for the stabilisation of nucleic acid from a biological sample, which comprises:
(a) collecting a biological sample;
(b) treating the sample so that a proportion of the 2', 3' or 5' -OH positions of the nucleic acid are modified with a protecting group; and
(c) subjecting the treated sample to one or more steps to isolate nucleic acid therefrom;
wherein the modified nucleic acid is subjected to a deprotection step comprising treatment with a primary amine to remove the protecting group.

In a second aspect, the present invention provides a kit for use in a method for the stabilisation of nucleic acid from a biological sample, which comprises:
(i) a reaction system for treating the sample so that a proportion of the 2', 3' or 5' -OH positions of the nucleic acid are modified with a protecting group;
(ii) an isolation system for subjecting the treated sample to one or more steps to isolate nucleic acid therefrom; and
(iii) a primary amine for subjecting the modified nucleic acid to a deprotection step to remove the protecting group.

In a further aspect of the invention there is provided a method for the isolation of a nucleic acid from a biological sample which comprises :
(a) collecting a biological sample;
(b) binding the nucleic acid to a solid phase in the present of an organic solvent;
(c) optionally washing the solid phase to remove contaminants; and
(d) eluting the nucleic acid from the solid phase; wherein the solid phase contains a metal or metal ion capable of coordinating with phosphate. In this aspect of the invention, the sample may or may not have been treated so that a proportion of the 2', 3' or 5' -OH, positions thereof are modified with a protecting group. Where this treatment has been made, the nucleic acid may be deprotected either before or after binding to the solid phase. The nucleic acid may be eluted from the solid phase using a chelator which may be subsequently removed using ultrafiltration, photosensitivity (where the chelator is photosensitive) or by affinity purification using an affinity tag on the chelator.

The proportion of 2', 3' or 5'-OH positions modified depends on the form and type of the nucleic acid to be modified. Whilst RNA contains 2', 3' and 5'-OH groups, DNA bears only 3' and 5'-OH groups. For RNA, a proportion is defined as at least one modification of the 2' positions, more preferably at least 10%, even more preferably at least 50%, even more preferably 75% and most preferably 100%. It is expected that for RNA modified at 5% or more of the 2'-OH positions, that at least one of the 3' or 5' hydroxyl positions will also be modified per molecule but this will also depend on whether the RNA bears a 5'phosphate rather than a 5'-OH group in which case the proportion will be lower. For DNA, a proportion can be defined as at least one 3' or 5'-OH group modification per molecule where the molecule can be either single or double stranded DNA.

The solid phase.is preferably hydroxylapatite, in which calcium ions are present. Other solid phases include iron (3) oxide and commercially available nickel-containing beads. Suitable metals include sodium, lithium, potassium, cesium, magnesium, titanium, chromium, manganese, calcium, iron, cobalt, nickel, copper, zinc, aluminium, silver, gold, platinum and lead, a metal oxide, a mixture of metals such as an iron-zinc blend or an oxide thereof, lithium iron III oxide, and ions thereof. The organic solvent is preferably water miscible and may be any one of the organic solvents described herein.
Suitable solvents include ; tetrahydrofuran, dioxolane, N-methyl pyrrolidinone (NMP), Formyl morpholine, Dimethyl imidazolidone, acetoxy acetone and acetonyl acetone. In a further aspect, the present invention provides a method for the stabilisation of nucleic acid from a biological sample, which comprises:
(a) collecting a biological sample;
(b) treating the sample so that a proportion of the 2', 3' or 5' -OH positions of the nucleic acid are modified with a protecting group; and
(c) subjecting the treated sample to one or more steps to isolate nucleic acid therefrom; wherein step (b) is carried out in a reaction medium which comprises an organic solvent having a flashpoint above 37°C, preferably above 60°C and more preferably above 90°C, which solvent is capable of forming a homogeneous solution with human blood when mixed in a ratio of 5:1 (vol:vol). Preferred solvents include N-methyl pyrrolidinone, formyl morpholine and dimethyl imidazolidone.

In a further aspect, the present invention provides a method for improving the template activity of a nucleic acid which comprises treating the nucleic acid with a metal ion chelator such as EDTA or EGTA to remove therefrom substantially all metal ions. Template activity includes reverse transcription using a reverse transcriptase such as, ARMY, MULV or TTh DNA polymerase or DNA polymerisation using a DNA polymerase such as Taq, Tth or Klenow fragment or RNA polymerisation using an RNA polymerase such as T3, T7 or SP6. The chelator is then removed from the nucleic acid by for example, ultrafiltration and the nucleic acid added to the polymerase reaction mixture whereupon the metal ions in the reaction bind to the phosphate of the nucleic acid. It has been found that nucleic acids treated in this manner have greatly improved template activity.

We have developed a method that provides nucleic acids of high purity and yield, but also in the case of RNA and DNA, are chemically protected from degradation prior to extraction thereby improving analytical precision. We have found that surprisingly, 2'-OH RNA is not only protected from nuclease, divalent metal cation and alkali degradation but also from freeze-thaw promoted degradation which is useful for RNA controls and standards which may be refrozen several times after an aliquot is used.

Described is a method for purifiying DNA and RNA from a sample, such as a biological sample or a clinical sample including cells, blood, serum and plasma. Advantageously, when the analyte is RNA, it is protected from degradation by chemical modification of the 2'-OH groups. The chemically protected RNA can be consequently deprotected using an organic primary amine which have been found to be particularly suited for this purpose. For both DNA and RNA purification, the method provides a means to stabilise nucleic acids in the sample, lyse cells and virus particles and remove proteins. Elution from the solid phase hydroxylapatite purification matrix is effectuated using a metal ion chelator such as EGTA.

### Deprotection of Nucleic Acids

Part of the invention relates to methods to remove acyl groups such as acetyl groups from acylated molecules such as 5' and/or 3' modified DNA and 2' modified RNA It is known that many if not all methods that have been described to remove, for example acetyl groups from acetylated compounds can lead to non-desired products along with the desired non-acetylated product. This is because the nucleic acid can be cleaved by the deprotecting compound. In this case the deprotecting compound is defined as a material that can remove an acyl group and in particular acetyl groups from an oxygen (acetyl) or nitrogen (acetamide) to restore the original hydroxyl or amide group respectively with only limited degradation of the nucleic acid itself One of the constraints of removing acyl groups from RNA and DNA polymerscompared with oligonucleotides is the increased probability of chain cleavage. A naturally occurring RNA polymer such as mRNA and viral RNA are on average 2,000-10,000 nucleotides in length. According to the present invention an oligonucleotide generally has a sequence length of up to about 80 bases and a polynucleotide generally has a sequence length of more than about 80, preferably more than about 100 bases. A preferred length for a polynucleotide is at least 1,000 bases. In order to remove acyl groups from a polymer compared with an oligonucleotide is technically difficult because of the increased risk that there will also be a non desired cleavage of the sugar-phosphate backbone. Degradation can be defined as cleavage of the sugar-phosphate backbone so that the 5' and 3' ends of the nucleic acid become separated and is particularly a problem when handling RNA. For a 2'-OH acetylated RNA molecule of, for example, 10,000 nt in length, it is desired that the number of sugar phosphate cleavages occuring during removal of the acetyl groups is less than 5%, more preferably less than 1%, even more preferably less than 0.01%.

The ease with which the acyl group can be removed from the acylated compound depends on a number of parameters that have been described extensively in « Protecting Groups in Organic Synthesis » Greene and Wuts, 2nd Edition, Wiley Interscience. In general, acyl groups are removed more easily from oxygen than from nitrogen. Another major factor is the group attached to the carbonyl, for example the formyl group (-CO-H) is simply removed at pH 9 and above, whilst the acetyl group (-CO-CH3) requires much harsher conditions to remove it. Longer chain lengths such as propanoyl (-CO-CH2-CH3) and butanoyl (-CO-CH2-CH2-CH3) are even more difficult to remove whilst substituted acyl groups such as methoxyacetyl (-CO-CH2-O-CH3), chloroacetyl (-CO-CH2C1) or trifluoracetyl (-CO-CF3) are much more readily removed than the unsubstituted acetyl. However, acetyl remains the predominately used acyl protecting group throughout industry, in part because it can be readily added to compounds from cheap and easily used reagents such as acetic anhydride, acetyl chloride and acetyl-imidazole. Furthermore, it has been found than when used in the presence of aqueous solutions such as blood, acetic anhydride is less liable to hydrolysis and therefore inactivation than reagents such as methoxyacetic anhydride or chloroacetic anhydride. Therefore it is necessary to use acylating reagents that are not so unstable in aqueous solutions that they hydrolyse before they can modify the nucleic acid, whilst modifying the nucleic acid with chemical groups that can be removed without leading to nucleic acid destruction. This is particularly problematic for RNA which is readily degraded by the types of alkalis that are efficient at removing the acyl groups. Methods of using such alkalis for the deprotection of the 2'-OH groups of RNA have been described elsewhere (WO/01/94626, WO/00/75302).

Numerous methods have been described to remove acetyl groups from acylated compounds including enzymatic, electrolytic and chemical means. Whilst enzymes such as esterases and lipases have found widespread use due to their mild reaction conditions, they are expensive, are often contaminated with non desired proteins or compounds and require careful preselection to find an appropriate activity. They are also sensitive to the charge on the acylated compound so that strongly charged molecules such as nucleic acids may not be good substrates. However, the use of suitable enzymes in particular esterases and lipases for deprotection of modified nucleic acids would be extremely useful.

Whilst many chemical deprotecting methods are also known for removing acetyl groups (Protecting Groups in Organic Synthesis, Greene and Wuts, 2nd Edition, Wiley Interscience) most if not all involve either a base or acid, conditions that are likely to lead to extensive cleavage of the desired RNA during deprotection.

Methods for protecting RNA by chemical modification have been extensively described in Patent application numbers WO/01/94626 and WO/00/75302. Fully or even partially acetylated modified RNA is protected from degradation from nucleases, however it is not capable of serving as a reverse transcription template and neither cannot it hybridise. Modified RNA can be stored, transported and archived in its protected form at ambient temperature instead of, as, is more usual in the industry, on ice, dry ice or in liquid nitrogen. Prior to analysis the modifications are removed. It is therefore important, after the RNA has been protected to be able to remove the acetyl groups in order to allow efficient reverse transcription, hybridisation and to serve as a template for protein synthesis. Methods to remove acetyl groups from RNA have been described in patent application numbers WO/O1/94626, WO/00/75302. These methods include the use of potassium cyanide, Hunigs Base, dimethylethylenediamine, sodium hydroxide and ammonium hydroxide.

Unfortunately, RNA is extremely sensitive to the presence of alkali and RNA chain cleavage occurs after the acetyl group has been removed from the 2'-OH position. It is therefore necessary to use a pre-calibrated amount of alkali sufficient to remove the acetyl groups from the RNA but not so much that it leads to significant subsequent RNA cleavage. Whilst this has been achieved by the use of mixtures of either sodium hydroxide or ammonium hydroxide with alcohol, some RNA chain cleavage is an inevitable result of acetyl deprotection using these methods (disclosed in patent applications; WO/01/94626, WO/00/75302). We have tested many other methods and chemicals have been tested for their activity to remove acetyl groups from RNA without leading to its cleavage including sodium hydrogen carbonate, sodium carbonate, potassium carbonate, potasium hydroxide, triethylamine, guanidine, hydrazine and HC1. These compounds either had no activity or lead to some degree of RNA chain cleavage and are therefore not the preferred method for deprotection of RNA although they are useful for the removal of acyl groups from DNA oligonucleotides and polynucleotides when the acyl group is either attached to the 5'-OH, the 3'-OH or the nucleobases. DNA is much less sensitive than RNA to being cleaved by alkalis. For example DNA can be incubated in 1M NaOH for one hour with no detectable degradation, conditions which would reduce RNA to monomers within 5 minutes. Others methods for the removal of protecting groups from the nucleobase of oligodeoxynucleotides but not the 2'- position of RNA polymers, that have been described in the literature as either mild or 'ultra-mild' deprotection methods (Glen Research, USA), are completely inappropriate for RNA because they would be expected to lead to extensive chain degradation. Furthermore, both methylamine and ammonium hydroxide which are widely used for are toxic and dangerous to work with and very strong smelling. They are not therefore suited to daily laboratory settings and must be used in a fume hood.

One published method (Boal et al., Nucleic Acids Res. (1996) 15:3115-3117) describes a method to deprotect benzoyl, isobutyrl or isopropoxyacetyl nucleobases on oligonucleotides following synthesis, employing gaseous ammonia at 10 bar pressure or methylamine at 2.5 bar in a pressured device. However, no mention is given of the utility of this method for removing modifications from the 2'- position of nucleic acids, rather the gaseous amines are used only for deprotecting the nucleobases of oligodeoxynucleotides. The method therefore does not mention deprotecting polynucleotides, RNA or the 2'-OH position all of which are useful to practice the invention as we have set out here. It is not expected that pressurised use of gases will find wide spread acceptance in laboratories. We have unexpectedly demonstrated that primary amines can not only remove protecting groups from the 2'-OH position of RNA but also lead to only limited cleavage of the phosphodiester backbone of RNA as would be expected for a base. It was also unexpected that primary amines such as ethylenediamine and triethylenetetramine reduce the amount of protein contamination binding to metal ions such as those present in hydroxylapatite.

It has been found that either modified RNA or RNA that has been immobilised on a solid phase such as charged nylon (Hybond N+, Amersham Biosciences, UK) and then treated with an alkali such as sodium hydroxide or ammonium hydroxide is more protected from subsequent alkali degradation than similar RNA in solution. This may be because the immobilised modified RNA or RNA has limited torsional freedom, that is the 2'-hydroxyl group in the presence of alkali and a solid phase cannot subsequently attack and cleave the 3' phosphodiester bond as would occur in solution. The cleavage effect of the alkali on the RNA is therefore reduced. Whilst this method is useful for deprotecting modified RNA for hybridisation purposes such as northern blotting, it has limited application for deprotecting modified RNA for reverse transcription or transcription mediated amplification (TMA) because polymerases such as reverse transcriptases cannot effectively copy immobilised RNA Methods for deprotecting modified RNA on solid phases have been set out in Patent application numbers (WO/O1/94626, WO/00/75302).

Other solid phases that provide only a temporary reversible binding of the modified RNA to the surface such as silica (Qiaex II particles (Qiagen, Germany) were found not to be useful for deprotection of RNA with aqueous alkalis because the RNA is eluted from the surface into the alkali during deprotection leading to its cleavage at normal rates. Other solid phases such as hydroxylapatite, although they bind RNA even in the presence of alkali, did not provide the level of protection from cleavage provided by charged nylon.

However, when acetyl modified RNA is bound onto a solid phase such as silica or hydroxylapatite it can be effectively deprotected without significant cleavage using dry gaseous ammonia. In this example, the solid phase may not be reducing the amount of cleavage, rather it may simply present the modified RNA to the ammonia such that a substantial proportion of the acetyl groups are readily accesible. By contrast, a precipitated or spin-dried pellet of RNA is less readily deprotected by ammonia gas possibly because of the difficulty of the ammonia entering the pellet and contacting all the acetyl groups. It is important that the ammonia vapour is dry because it has been found that the presence of water leads to RNA cleavage.

Pressurised bottled ammonia is a suitable dry source or alternatively, ammonia can be conveniently distilled from a solution of 28% ammonium hydroxide. In the latter example, it is important to pass the ammonia vapour over at least one surface to condense any water vapour that may have been carried over with the ammonia from the ammonium hydroxide solution. The dried ammonia can then be passed into a flask containing one or more tubes bearing the modified RNA bound onto beads. Deprotection times vary from a few minutes to one hour, depending on (i) the concentration of ammonia, (ii) the temperature, and (iii) the amount of RNA exposed to the ammonia in the particle pellet. It has been found that dense pellets of silica or hydroxylapatite bearing the modified RNA are deprotected more slowly than diffuse pellets of particles. Controlling the pellet size can be difficult and therefore it can be difficult to judge the necessary deprotection time. Deprotected RNA can be simply washed to remove traces of the by product ammonium acetate and eluted for downstream applications. Unfortunately this method is somewhat cumbersome and unpredictable for routine use and involves working in a high performance chemical fume hood.

An improved method of deprotection has been developed that does not involve gaseous materials. Although the use of methylamine (CH3-NH2) is known for the removal of acryl groups from nucleobases of oligonucleotides, it is a dangerous, gaseous,toxic chemical to use which would have unkown effects on fragile molecules such as polymers in particular RNA polynucleotides. One of the problems is that methylamine is its low boiling point (-6°C) and a high vapour pressure (2250mm Hg at 20°C) so that evaporation rates are very high indeed. Similarly ammonia boils at -33°C and has a high vapour pressure making its use dangerous. Both ammonia and methylamine are gases at room temperature and pressure. This, preferred method involves the use of primary amine compounds with vapour pressure below 2000mm Hg, more preferably below 1000mmHg, even more preferably below 500mm Hg and most preferably below 200mm Hg at 20°C. This preferred method involves the use of primary amine compounds with boiling points above 0°C, more preferably above 25°C, even more preferably above 50°C and most preferably above 100°C at 1 atmosphere of pressure.

Long chain (containing five carbon atoms or more) primary amine compounds such as pentylamine have boiling points above 100°C, however, the rate of deprotection with these compounds is expected to be slower than for shorter (less than five carbon atoms) chain primary amines such as butylamine because of steric hinderance between the nucleic acid and the bulky long chain amine. There is therefore a trade-off between the rate of deprotection of the primary amine and it's boiling point/ volatility. However, advantageously, compounds that can extensively hydrogen bond such as ethanolamine are not only relatively smalls molecules thereby reducing steric hinderance but they also have boiling points above 100°C because of hydrogen bonding. Therefore primary amine containing compounds that can also hydrogen bond extensively are both useful because they deprotect quickly but also because they have significantly lower vapour pressures so they are easy to handle. Molecules with two or more amine groups such as ethylenediamine are preferred to monamines such as ethyleneamine because of their greater reactivity, higher boiling and flash points and lower vapour pressures.

Examples of suitable primary amine compounds for deprotection include ethanolamine (Fluka, USA), ethylenediamine (Fluka, USA), diethylenetriamine (Fluka, USA), triethylenetetramine (Fluka, USA), tetraethylenepentamine (Fluka, USA). Other primary amines include diglycolamine agent (Huntsman, USA), Jeffamine (polyoxyalkyleneamine) type molecule including Jeffamine ED which is water soluble (Huntsman, USA), dimethylaminopropylamine (Huntsman, USA) and methoxypropylamine (Huntsman, USA).

It has also been found that other primary amine containing compounds such as the amino acids Lysine and Arginine are good reagents for the deprotection of acylated nucleic acids, in particular acylated RNA. These amino acids contain both the α-NH2 and the ε-NH2 groups, both of which, in the free base form, may contribute to the removal of acyl groups. Advantageously, these amino acids compounds have a very low volatility, are relatively cheap, widely available and create few disposal problems as they are biodegradable. Indeed they are excellent 'green chemistry' solutions. Lysine and arginine can be purchased either in their free base form or as their salts, the free base forms are preferred.

Such deprotection reagents as ethanolamine, ethylenediamine, diethylenetriamine, triethylenetetramine, lysine and arginine may also be useful for removal of unwanted modifications of RNA and DNA made during the use of diethyl pyrocarbonate (DEPC), a widely used inhibitor of RNases. The non desired DEPC modifications of nucleic acids for example on the nucleobases, lead to reduced nucleic acid template efficiency, therefore their removal is desired if full nucleic acid template activity is to be restored.

Advantageously it has been found that deprotection can be carried out whilst the RNA is immobilised on a solid support, particle or bead or other solid phase such as hydroxylapatite or silica. The solid phase may consist of an organic or inorganic particle, a polymeric linear, globular or cross-linked molecule or resin. It may be made of a variety of materials or material composites such as acrylamide, agarose, cellulose, polyamide, polycarbonate, polystyrene, polyethylene, polypropylene polytetrafluoroethylene, nitrocellulose, latex, aluminium, copper, nickel, iron, a metal oxide, a mixture of metals such as an iron-zinc blend or an oxide thereof, lithium iron III oxide, glass, hydroxylapatite or silicon.

The solid phase may also be a composite between a particle or surface and an immobilised nucleic acid, in particular an oligonucloetide complementary to the desired target analyte nucleic acid. Preferably the solid phase bearing the complementary oligonucleotide is a magnetic particle thereby aiding handling during purification. In order to allow effective capture of an analyte RNA using a complementary oligonucleotide, the RNA must be at least partially deprotected prior to hybridisation, because acetylation of RNA at the 2'-OH position reduces or abolishes the capacity of the RNA to hydrogen bond with a complementary oligonucleotide. It is particularly convenient to protect the analyte RNA molecule and then to deprotect and capture it with a complementary oligonucleotide in the presence of a primary amine deprotection reagent.

The solid phase may also possess specific properties that aid in the manipulation of the particle such as paramagnetic or magnetic properties, a diameter allowing retention by a filter, an increased density that enhances sedimentation or separation by centrifugation or incorporate a tag aiding identification, capture or quantification of the particle. This solid phase provides a simple means to separate the deprotection reagent from the nucleic acid sample after deprotection. However, primary amines such as ethanolamine, ethylenediamine, diethylenetriamine, triethylenetetramine, lysine and arginine can be used to deprotect the RNA when the RNA is either (i) attached to a solid support (see example 23), or (ii) in solution (see example 1).

Primary amines for use in this invention include; C1-C10 alkylamines, C1-C10 aminoalkylamines, C2-C10 hydroxyalkylamines, C2-C10 haloalkylamines, C4-C10 di(aminoalkyl)amines, C4-C20 dialkylaminoalkylamines, C4-C20 alkyloxyalkylamines and C3-C10 diaminocarboxylic acids

Very usefully, it has been found that primary amines such as ethanolamine, ethylenediamine, diethylenetriamine, triethylenetetramine, lysine and arginine not only serve as useful deprotection reagents but they also reduce the amount of non-desired proteins that bind to hydroxylapatite and silica during nucleic acid purification. Addition of 200µl of ethylenediamine to a 1.45ml reaction containing 200µl of plasma, 50µl of 1-methylimidazole and 1.2ml of tetrahydrofuran / acetic anhydride (2:1 vol :vol) has been found to reduce protein binding by 7.5 fold whilst not affecting RNA yield. This is an unexpected result because hydroxylapatite and silica surfaces are well known to bind proteins. This highly desirable property of primary amines and to a lesser extent non-primary amines such as triethylamine, pyridine, TEMED, dimethylpropylamine and dimethylethylenediamine can be used to reduce the amount of proteins binding to the solid phase during purification of RNA and DNA from a biological sample. It is known that ethylenediamine and triethylenetetramine can bind to metal ions by coordination and it is perhaps this 'chelating' to calcium on the hydroxylapatite that partly explains the reduced protein binding. It is preferable, but not essential to add the amine premixed with the hydroxylapatite prior to addition to the sample, but no advantage was noted by incubating the reaction with ethylenediamine for periods longer than 10 min. prior to adding the hydroxylapatite.

Hydroxylapatite binds to charged biomolecules by a combination of its positively charged calcium ions, negatively charged phosphate ions and hydroxyl groups. Although nucleic acids such as RNA and DNA carry a strong negative charge allowing binding to the hydroxylapatite calcium ions, so also do many proteins. Furthermore, many proteins are also positvely charged so that they bind to the phosphate groups of the hydroxylapatite. Frequently, the nucleic acid is in very low concentration compared with the contaminating proteins, lipids and carbohydrates present in the cell or biological sample such as blood, serum etc. For example, blood contains approximately 60 000 times (w:w) more protein than RNA. As a result it can be difficult to purify the nucleic acid away from the contaminants using hydroxylapatite. Most methods have relied on using differential elution to separate the nucleic acids from the contaminants, however, such preparations are either low in the desired nucleic acid or contain significant amounts of the non desired contaminant. The contaminant may reduce the efficiency of downstream applications of the nucleic acid such as reverse transcription, PCR, hybridisation or even lead to its degradation if significant amounts of nucleases are eluted with the nucleic acid analyte.

Therefore, inhibiting proteins from binding to the hydroxylapatite whilst still allowing deprotection and nucleic acid binding to occur is highly advantageous. We have found that purification of nucleic acids, in particular RNA from serum and plasma has been found to be improved by the addition of the deprotection amine reagent to the chemical modification reaction bearing the acylated RNA. For the purification of modified RNA from biological samples such as blood plasma without deprotection occuring but still inhibiting proteins from binding the purification matrix such as silica or hydroxylapatite can be accomplished by using secondary and tertiary amines. Secondary and tertiary amines have been found to inhibit proteins from binding to silica and hydroxylapatite but do not lead to deprotection of the RNA. Therefore, in this example, modified RNA can be purified from complex, biological samples by the addition of a secondary or tertiary amine to the reaction before, or at the same time as the purification matrix is added to the mixture. The modified RNA can then be purified and eluted from the matrix in its protected form. The modified RNA can also be stored and archived in its protected form and deprotected using a primary amine immediately prior to use for example hybridisation or RT-PCR.

Likewise, DNA can be separated from contaminating proteins by the use of primary, secondary and tertiary amines in conjunction with the solid phase matrix such as silica or hydroxylapatite beads. The purpose of the amine in this case is not only to remove any potential acetyl groups attached to the DNA, for example at the 5' and 3' OH positions but also to inhibit the binding of proteins to the purification matrix.

Modified RNA can be deprotected at any one of three points during purification (i) at the same time as binding to the solid phase purification matrix as described in example 1, (ii) after the modified RNA has been been bound to the solid phase purification matrix, but before elution, and (iii) after elution from the solid phase purification matrix. The advantage of (i) is that the modified RNA is deprotected and proteins removed at the same time, the advantage of (ii) is that the deprotected RNA can be readily removed from the deprotection reagent by means of the solid phase it is bound to, whilst the advantage of (iii) is that the RNA is purified in its modified and therefore protected form. However, with method (iii) there is a necessity to separate the deprotected RNA from the deprotection reagent. This can be achieved by filtration using a Centricon device (Millipore, USA) according to maufacturer's instructions, precipitation using alcohol or dialysis. Alternatively, the RNA can be separated by evaporating the deprotection reagent away using reduced pressure or increased temperature. In this case, a deprotection reagent with a decreased boiling point is preferable to one with a high boiling point. Primary amine reagents with a reduced boiling point are propylamine (bp 47°C) or butylamine (bp 76°C). Alternatively, the deprotected RNA can be removed from the deprotection reagent by binding it onto a solid phase deprotection reagent such as hydroxylapatite, washing the beads uding 70% ethanol to remove excess deprotection reagent, and then eluting the RNA using phosphate or a chelator.

The amine may also be immobilised on a solid phase so that conveniently the deprotection reagent on for example a bead, can be mixed with the protected RNA, deprotection allowed to proceed and then it can be removed from the deprotected RNA on the basis of a property of, the solid phase. This property can be a paramagnetic core or its collection during centrifugation or filtration. Suitable materials include ethylenediamine polymer bound (Aldrich, USA catalogue number 47,209-3)

The use of magnetic or paramagnetic preparations of hydroxylapatite or even silica is preferred because of the ease of handling, washing and the large surface area of particles compared with other forms of the solid phase such as a membrane.

### Solvent

An alternative solvent to tetrahydrofuran for the modification of nucleic acids, in particular RNA, is dioxolane, a water miscible solvent with a lower vapour pressure and higher boiling point than tetrahydrofuran. However, dioxolane like tetrahydrofuran, produces potentially dangerous flammable vapours. Other solvents with much lower vapour pressures and much higher flash points that have been found to be excellent alternatives to either dioxolane or tetrahydrofuran, include N-methyl pyrrolidinone (NMP), Formyl morpholine, Dimethyl imidazolidone, acetoxy acetone and acetonyl acetone. Solvents are preferred with flash points above 37°C, more preferably above 60°C and even more preferably above 90°C. Particularly preferred are N-methyl pyrrolidinone, Formyl morpholine, Dimethyl imidazolidone, their physcial properties compared with tetrahydrofuran are set out in Table 1. Surprisingly, these three solvents have a higher capacity to dissolve complex biological samples such as blood and plasma proteins than does either tetrahydrofuran or dioxolane so that less precipitates form during handling of mixtures of biological samples and organic mixtures, thereby increasing nucleic acid yield and reducing protein contamination of the nucleic acid. For example 200µl of blood can be dissolved to form a homogenous mixture in 1ml of N-methyl pyrrolidinone whilst the same amount of blood mixed with either tetrahydrofuran or dioxolane causes the proteins to rapidly precipitate out of solution. N-methyl pyrrolidinone, Formyl morpholine and Dimethyl imidazolidone may find other uses in life sciences for dissolving for example recombinant therapeutic proteins that are insoluble when released using traditional methods from cells or cell free translation systems. Both tetrahydrofuran and dioxolane require the presence of approximately 5% of 1-methylimidazole in order to dissolve up to 20% final volume of human plasma, whilst N-methyl pyrrolidinone, Formyl morpholine, Dimethyl imidazolidone can completely solubilise this amount of plasma without any addition of 1-methylimidazole. Advantageously, N-methyl pyrrolidinone is biodegradable improving disposal issues.

**Table 1. Comparison of the properties of various solvents**

| **Solvent** | **Flash point** | **Water solubility** | **Other properties** |
|---|---|---|---|
| Tetrahydrofuran | - 17°C | complete | - |
| Dioxolane | - 3°C | complete | - |
| N-methyl pyrrolidinone | 91°C | complete | biodegradable |
| Formyl morpholine | 125°C | complete | Compatible with polycarbonate |
| Dimethyl imidazolidone | 102°C | complete | |

### Reactant

It has been found that acrylic anhydride, propionic anhydride or butryic anhydride (Fluka Chemicals, USA) can replace acetic anhydride in example 1. The advantages of these reagents is that they may have longer shelf storage lifetimes compared with acetic anhydride and they may also be more stable when premixed with the solvent and catalyst as set out below. These reactants have been described in (WO/01/94626 and WO/00/75302).

The reactant, catalyst and solvent can be predispensed in a blood collection tube so that the blood sample is drawn directly from the patient or blood collection pouch into the tube where it contacts the reactant and the nucleic acids are stabilised. The blood collection tube containing the stabilised nucleic acids can then be transported, archived or used directly to extract and purify the nucleic acids. The advantage of this approach is that a single tube contains all the necessary reagents necessary for nucleic acid stabilisation, thereby minimising the manual steps required to initiate the stabilisation reaction. In one manifestation of the device, blood is drawn directly from the vein of the patient, via a needle and tube into the appropriately marked tube containing both the necessary reactants and a vacuum sufficient to induce blood flow into the tube. In another format, blood is drawn from the patient using a syringe and needle and then injected by way of the needle and a rubber septum at the top of the tube, into the reactant. Complete mixing is assured by gentle inversion of the tube. The tube and septum or top of the tube are preferably composed of materials that are chemically compatible with the reagents, such as those composed of polypropylene, polyethylene, glass or PTFE. Such a device is particularly suited for diagnostic purposes for example of, viruses such as HIV and HCV.

### Catalysts

The advantage of 1-methylimidazole as an acylation catalyst is that it also serves as a very effective buffer so that the acidification of the reaction caused by the accumulation of acetic anhydride is buffered. Additionally, 1-methylimidazole is an excellent solvent for dissolving biomolecules such as proteins. However, surprisingly, other catalysts have been successfully used including 4-pyrrolidinopyridine and 2-hydmxypyridine as set out in example 31. However, it has been found that whilst a mixture of either 1-methylimidazole, N-methyl pyrrolidinone and acetic anhydride or 4-pyrrolidinopyridine, N-methyl pyrrolidinone and acetic anhydride retains its acylating activity after storage for 11 days at ambient temperature, the mixture 2-hydroxypyridine, N-methyl pyrrolidinone and acetic anhydride loses its activity after five days. Further comparison are set out in example 30. Therefore the use of the catalysts 4-pyrrolidinopyridine and 1-methylimidazole is preferred for storage of mixtures containing acylating reagents. These catalysts have been described in (WO/01/94626 and WO/00/75302).

### Volumes of reagents

For the purification of nucleic acids from a 200µl plasma or serum sample according to example 1, 1.25ml of organic reagents (catalyst, solvent and reactant) are added to the sample. It has been found that only 1.05ml of organic reagents are necessary for the modification, stabilisation and purification of the RNA. The reduced reaction volumes also means that less deprotection reagent volumes can be added, the volumes of the deprotection reagents can be reduced from 3.4ml to 2.6ml as set out in example 27 below.

It has surprisingly been found that the nucleic acid analyte can be bound onto the hydroxylapatite beads prior to the application of the deprotection agent as set out in example 28, The advantage of this approach is that a smaller volume of deprotection reagent can be added to the hydroxylapatite/ RNA complex because it does not become quenched by the acetylating reagent present in the reaction. In example 1, a relatively large amount of both 1-methylimidazole and ethylenediamine are added at the same time as the hydroxylapatite beads because the acetic anhydride/ acetic acid present in the reaction reacts with these components. Using the method as set out in example 28, the hydroxylapatite beads are first added to the reaction, and following binding of the RNA to the beads the reaction containing the acetic anhydride/ acetic acid can be removed, the beads washed and then a small amount of deprotection reagent added. It has been found that the volume of the deprotection reagent required is reduced from 3.4ml to 0.2ml or less.

It has been found that addition of 10µg of carrier RNA in the reaction improves the RNA yield 1.3 fold. This is probably due to suppressing non-specific interactions between the RNA anlayte and plasticware. Both poly rC (Midland Certified Reagent Company. USA) and total yeast RNA had similar effects. Carrier RNA also improved the recuperation of the analyte RNA following ultracentrifugation using a Microcon-YM-50 device by 1.25 fold.

### Hydroxylapatite beads

Hydroxylapatite magnetic beads are commercially available (Chemicell, Germany), however, due in part to the organic reagents and in part to the density of the liquids with which the beads are mixed, complete bead collection using a fixed magnetic stand (Promega, USA) is less than complete. It has been noted that a proportion of the HXA Type I bead population is composed of much smaller particles, that nevertheless bind a significant proportion of the desired nucleic acid in the sample. The loss of these smaller particles represents an important reduction in the overall yield of nucleic acid. Complete collection of all particles can only be achieved by centrifugation of the solution containing the particles at 14 000 x g for 5 minutes or filtering the mixture using an Acrodisc GHP (13mm) and a syringe (Catalogue number PALL 4556, Pall Inc, USA). It has been found that by using this Acrodisc filter that the overall RNA yield increases from 80 to 100%. In order to avoid the use of filters, hydroxylapatite beads were selected with an increased content of larger particles that were more eaily collected using a magnet as follows. 1ml of hydroxylapatite particles was added to 50ml of 80% glycerol, mixed, then centrifuged at 3000 x g for 10 minutes. The supernatant containing the smaller particles was discarded and the pellet washed in 20 ml of water and resuspended in 1 ml of water. Hydroxylapatite beads prepared in this way are more effectively collected by magnetic collection than the non-size selected beads. In general, magnetic-hydroxylapatite beads are prepared that (i) efficiently bind nucleic acids with relatively little protein contamination, (ii) can be collected easily using a fixed magnet, (iii) do not disintegrate or dissolve in the presence of organic or aqueous solvents or reactants and (iv) readily release the nucleic acid on application of the elution solution and (v) allow the differential release of RNA, DNA and proteins in distinct fractions for subsequent analysis of each fraction.

It has been found that for magnetic-hydroxylapatite bead type 14/2 (Chemicell, Germany), the optimum amount of beads for RNA purification is 25µl (50mg/ml). Addition of more beads had the effect of reducing RNA yield whilst increasing significantly the protein contamination.

### Pretreatment of magnetic hydroxylapatite

It has been found that pretreating hydroxylapatite beads (Chemicell, Germany) with 0.2M sodium phosphate (Sambrook et al., (1989) Molecular Cloning: A Laboratory Manual, CSH.) before use, as set out in example 1, improves the ratio of RNA to protein bound to the beads. This is probably because the sodium phosphate reduces the overall charge on the surface of the beads thereby favouring the binding of more highly charged molecules such as nucleic acids over less charged such as proteins thereby increasing the yield and purity of the desired nucleic acid. Whilst sodium phosphate is effective in this function, organic phosphates such as 0.2M glucose phosphate, cellulose phosphate or serine phosphate (Sigma-Aldrich, USA) did not have the desired effect of increasing nucleic acid yield and purity, rather, RNA yield was decreased compared with sodium phosphate treated hydroxylapatite. However, protein contamination was also reduced suggesting that it may be possible to pre-treat the beads with a phosphate that has the desired property of reducing protein contamination whilst increasing RNA yield. It is expected that other soluble phosphates such as potassium phosphate would have a similar desirable effect to sodium phosphate. Examples of potentially useful compounds include D-arabinose-5-phosphate, D-6-Fructose-phosphate, D-glucosamine-6-phosphate, D-mannose-6-phosphate, D-ribose-5-phosphate, D-ribulose-5-phosphate, D-glyceraldehyde-3-phosphate, D-sorbitol-6-phosphate, DL-ascorbic acid-2-phosphate, glycerol phosphate, ammonium phosphate, barium phosphate, bismuth III phosphate, boron phosphate, citrate phosphate, cobalt phosphate, copper II phosphate, threonine phosphate, nucleotide monophosphate, nucleotide diphosphate and nucleotide triphosphate.

### Types of nucleic acid binding solid phases

Although hydroxylapatite is efficient at binding nucleic acids from an organic-aqueous mixture, other types of solid phases can also be used. These include silica such as Qiaex II (Qiagen, Germany), derivitised silica such as Magnisil (Promega, USA) and surprisingly simply iron III oxides (<5µm particle size) (Aldrich, USA, catalogue number 31,006-9). Nucleic acids can be removed from iron oxides by mixing the particles with 0.1M sodium phosphate buffer (pH 6.8) or alternatively using 100mM EGTA-TEAH salt (pH 10.2). It has been found that Iron III chloride, 5 wt % on silica gel (Aldrich, USA, catalogue number 36,100-3) and large pieces of Iron III oxide (>2mm diameter pieces) are both very effective at binding nucleic acids from both aqueous and organic reactions. Elution can be brought about by the addition of 0.2M Sodium phosphate or 10mM EGTA, pH 10.2.

### Elution and removal of chelator

In the preferred method of practising nucleic acid purification, as set out for example, below, nucleic acids are eluted from magaetic-hydroxylapatite using a chelator such as EGTA, EDTA, DTPA, HEDTA, NTA and BAPTA. However, the presence of the chelator with the desired nucleic acid can inhibit the activity of enzymes requiring divalent metal cations such as Tth DNA polymerase. These enzymes are necessary for downstream analysis of the nucleic acid. Therefore the chelating activity is desired for elution but undesired following elution. The chelating activity present with the nucleic acid solution therefore needs to be removed. A minimum amount of the chelator should be used that is necessary for elution of the desired analyte so that only a minimum is present with the eluted nucleic acid.

It has suprisingly been found that the type of salt of the chelating agent is extremely important for the chelating activity and hence the capacity to release the nucleic acid from the magnetic-hydroxylapatite. For the salts tested, sodium and potassium salts of EGTA were the least effective whilst, ammonium salts, prepared using ammonium hydroxide addition to the free acid form of EGTA, was significantly better. The most effective salts of EGTA were found to be tetramethylammonium, tetraethylammonium and tetrabutylammonium at pH 9.9 all of which were approximately equivalent. These salts were approximately 1.9 fold more effective than the ammonium salt of EGTA at releasing RNA from magnetic-hydroxylapatite. Tetraalkylammonium salts of chelators such as for example, EDTA, NTA, BAPTA and EGTA may be useful for other applications requiring strong chelators.

The pH of the elution solution also has a profound effect on its capacity to release the nucleic acid from magnetic-hydroxylapatite. Whilst for example, a solution of 10mM EGTA pH 8 was poor at releasing a 32P labelled RNA sample from magnetic-hydroxylapatite, there was an improvement, compared with 10mM EGTA (pH 9), of 1.12 fold (pH 9.3), 1.4 (pH 9.6), 1.5 (pH 9.9) and 1.45 (pH 10.2). The optimum pH was therefore determined to be approximately pH 9.9. The use of chelating solutions that have maximum activity is critical for reducing the elution volume to a minimum. For example, using a solution of 10mM EGTA pH 8.7 (sodium salt), the minimum volume for complete RNA elution was found to be 400µl, whilst for a solution of 10mM EGTA pH 8.7 (tetraethylammonium salt) the volume could be reduced to 50µl.

One method for chelator removal is ultracentrifugation using filter units with MWCO of 5 000 to 100 000 daltons. Processing of samples therefore requires centrifugation as set out in example 1 and is significantly faster than dialysing solutions to remove the chelator. However there are alternative methods for removing the chelator from the RNA as set out below.

Methods for removing the chelating activity include using photo-sensitive chelating reagents (so called 'caged calcium' reagents) such as NITR-5, NTIR-7 (U.S. Patent 4,689,432 and 4,806,604), nidophenyl-BAPTA (Graham et al., (1994) Proc. Natl. Acad. Sci. 91 :187), NP-EGTA and DMNP-EDTA. These reagents are photo-sensitive and destruct on exposure to short bursts of light of the correct wavelength. Therefore the non-desired chelating activity can be destroyed by subjecting the eluted nucleic acid/ chelator solution to light of the appropriate wavelength and exposure. For example, NP-EGTA has a 12 000 fold reduction in its affinity for calcium on exposure to uv light. Methods, materials and references describing the use of some of these reagents for the release of intra-cellular metal ions have been described (Handbook of Fluorescent Probes and Research Chemicals, Chapter 20, Molecular Probes). Nucleic acid - photosensitive chelating mixtures could be exposed individually or en masse to the light source whilst present in microcentrifuge tubes or 96 well plates. The photo-destruction of the chelator should be achived with the smallest amount of light necessary to achieve sufficient cleavage so that minimum photo-destruction occurs to the nucleic acid analyte.

An alternative use of photosensitive chelators is as follows. Many important enzymatic reactions such as PCR and reverse transcription require divalent metal cations, in particular Mg and Mn for activity, however premature activity of these enzymes particularly at a reduced temperature can lead to non-specific polymerisation products. Several methods have been employed to reduce premature polymerisation occurung including so-called 'hot-start' methods where the enzyme is added to the reaction only after the apprpriate temperature has been reached, or the enzyme only becomes active at the correct temperature. However these are either likely to increase contamination or are expensive. Alternatively, a photo-sensitive chelator could be added to the reaction at a concentration equal to the amount necessary to remove all the divalent metal cations present, thereby inhibiting the enzyme activity. Once the correct reaction temperature has been obtained, the reaction can be exposed to an appropriate light source thereby destroying the chelator and releasing the divalent metal cation and initiating the polymerisation reaction. The light source could be for example one already located in the amplification machine (LightCycler, Roche, USA) or an external source.

Another method for removing chelating activity is to use an antibody specific for the chelating agent, for example anti-BAPTA IgG (Cell Calcium 21 :175 (1997) ; Cell Calcium 22 111 (1997) which is commercially available (Molecular probes Inc, USA). In this example the elution solution would be BAPTA, and the anti-BAPTA antibody would be tethered directly or via secondary antibody to a solid phase such as protein A-sepharose (Sigma-Aldrich, USA) and the elution sulution incubated with the antibody allowing the chelator but not the nucleic acid analyte to be removed.

Yet another method to remove the chelating activity would be to use a 'tagged' chelator. The tag could be for example, a biotin molecule attached to the chelator DTPA. Following elution, the chelator is removed from the nucleic acid using streptavidin or an avidin solid phase that are commercially availbale from a number of vendors (e.g. Dynabeads M-280, Dynal, Norway). Methods for attaching the chelator DTPA to other molecules has been described (Hnatowich (1983), Science 220 :613) and materials are commercially available such as EDTA maleimdo-C5-benzyl (Calbiochem Inc, USA) or biotin-chelator is commercially available as biotin-DTPA (catalogue number D1534, Sigma-Aldrich, USA).

### Magnetic collection

We have found that using an external magnet such as those commercially available (Promega, USA, Dynal, Norway) offer an effective method to collect large magnetic or paramagnetic beads from solution. However, when the liquid containing the magnetic beads is viscous and /or a volume greater than 5ml, the rate of bead collection is reduced leading to losses. An alternative to an external magnet is the use of a disposable magnet that is added directly into the tube containing the magnetic beads. The distance between the magnet and beads is therefore markedly reduced thereby increasing the magnetic force exerted on the magnetic beads and improving bead collection. Suitable types of disposable magnets are those that have a non-reactive non-contaminating surface such as PTFE. These are commercially available as magnetic stirring bars used to dissolve solids into liquids. Examples of products include Aldrich catalogue numbers; Z42,022-0, Z32,866-9 and Z32,868-5 all of which are relatively cheap devices. The shape of the magnetic stirrer is not particularly limited, but those with sufficient surface area to bind all the beads in the liquid without the beads overlapping one another are preferred. It has been found that a 2cm long, 6mm diameter stirrer offers more than sufficient surface area to effectively bind 2.5mg magnetic hydroxylapatite Type I (Chemicell, Germany). Usefully; the magnetic stirrer after it has been added to the liquid containing the beads can be manipulated in a sealed tube by the use of an external magnet, so for example, it can be lifted out of the liquid, allowing the liquid to be removed and the stirrer washed by the addition of fresh reagents. Surprisingly, the magnetic beads can be temporarily removed from the stirrer by gently vortexing the tube containing the stirrer, beads and a liquid, allowing efficient washing of the beads to occur. The stirrer could have a central hole allowing a disposable plastic pipette tip to insert through it and remove liquid around and beneath the stirrer thereby improving the dispensing and removal of for example, wash solutions. Low protein binding surfaces such as PTFE or polypropylene are preferred because they reduce protein contamination and are compatible with solvents and reactants such as tetrahydrofuran and acetic anhydride.

It has also been found that magnetic-hydroxylapatite beads can be quickly collected by placing two electrodes in the solution containing the beads and connecting the leads to a 9 volt battery. The magnetic-hydroxylapatite beads collected on both the anode and cathode completely coating the surfaces. The beads could then be released from the electrodes for washing or elution by turning off the current. The efficiency of the collection be be improved by the addition of an electrolyte such as 0.5X TAE electrophoresis buffer.

### Magnetic mixing

It has been found that specialised magnetic mixers such as the MCB1200 (Dexter Magnetic, UK) are effective for agitating solutions containing magnetic beads. However, it is relatively expensive device and is limited to 12 available tube spaces. Surprisingly it has been found that magnetic stirrers designed for use with magnetic stirring bars are also extremely effective at agitating magnetic beads in a liquid enclosed within for example, a 1.5ml polypropylene microcentrifuge tube. The microcentrifuge tube is simply stood, or laid flat on the top surface of the magnetic stirrer and the speed setting adjusted to between 100-900rpm. The beads are vigorously agitated within the microcentrifuge tube, which itself is immobile. A simple microcentrifuge tube holder made of foam or plastic and set on top of the magnetic stirrer provides a convenient means to hold the tubes at the same distance from the rotating permanent magnet underneath. The beads can be collected by moving the tube holder containing the tubes to a fixed magnetic stand. Suitable magnetic stirrers include IKA® magnetic stirrers (Aldrich, catalogue number Z40,482-9, Z40,372-5). Alternatively, magnetic stirrers with no electric motors but rather, a series of oscillating electromagnets can be used effectively to thoroughly mix the magnetic beads (Aldrich, catalogue number Z31,233-5).

### Example 1

### Purification of RNA from human blood plasma

Various types of RNA can be purified from plasma such as the clinically important viruses HCV and HIV. The virus capsids are disrupted in the presence of 1-methylimidazole, tetrahydrofuran (THF) and an acylating reagent such as acetic anhydride. This mixture also leads to the disruption of the nucleoprotein complex and consequent release of the RNA which can then be chemically modified and stabilised.

Stabilisation of the RNA analyte: To 200µl of human plasma (EDTA coagulation inhibitor) or serum in a 15ml screw top polypropylene centrifuge tube (Falcon, USA) was added 50µl of 1-methylimidazole, the mixture was mixed briefly and then 600µl of a mixture of tetrahydrofuran and acetic anhydride (2:1 vol/vol) was added and mixed by gentle pipetting with a 1ml pipette tip. A second addition of 600µl of tetrahydrofuran and acetic anhydride (2:1 vol/vol) was added within 1 minute of the first addition and the solution mixed again. Other types of acylating reagents such as propionic, butanoic, pentanoic, heptanoic or benzoic anhydrides can be used as alternatives or in conjunction with acetic anhydride. Acylation reagents and methods for chemically modifying RNA have been described (WO/01/94626 and WO/00/75302)

Following addition of the acylation reagent, an internal control such as one composed of RNA (e.g. 8µl of the HCV Internal Control, version 2.0, Roche Diagnostics, USA) or DNA or even a bacteriophage (Armoured RNA, Hepatitis Virus Control, Ambion RNA Diagnostics, USA) may be added. Following an incubation for 10 minutes at 37°C, the solution containing the stabilised RNA can be stored for prolonged periods at up to 37°C. The RNA can also be transported and handled in its protected form. Alternatively, the RNA can be purified immediately following the 10 minute incubation at 37°C as described below.

It has been found that with human plasma samples such as those originating from blood donations, on addition of tetrahydrofuran and acetic anhydride, the reaction turns bright fluorescent yellow, serving as a useful indicator that the reaction was successful. On standing for 10 minutes the reaction turns brown.

Processing of the stabilised RNA: Following stabilisation of the RNA during which the RNA sample can be either stored, transported or processed immediately (following the 10 minute incubation at 37°C) the RNA is separated from the reaction and contaminants by differential binding to a solid support such as silica or hydroxylapatite in the presence of an organic amine. Alternatively, but less preferably the RNA can be purified by precipitation, dialysis or spin column filtration. The RNA can also be spotted and immobilised directly onto a solid support such as nylon (Hybond N+, AmershamPharmacia Biotech, UK) and analysed by hybridisation with a labelled complementary probe.

It has been found that primary amines such as ethylenediamine or ethanolamine are particularly suited to reducing contamination by protein binding to the solid phase used to bind and purify the nucleic acid, and in the case of RNA, also leads to the cleavage of the protecting acetyl group from the 2'-OH position of the RNA. The primary amine therefore has two useful properties (i) reducing protein contamination and, (ii) deprotecting the RNA without leading to consequent phosphodiester cleavage. The use of ethylenediamine and ethanolamine for the deprotection of oligonucleotides has been described (Miller, P.S. et al. (1986) Biochem. 25:5092; Hogrefe et al., Nucleic Acids Res (1994) 22:5492 ; Polushin, N. (1994) Nucleic Acids Res. 22 :639; Polushin, N. (1991) Nucleic Acids Symp Ser. 24 :49). However, the use of these deprotection reagents was limited to the removal of protecting groups from the nucleobases of DNA and it is unexpected that our results have demonstrated that primary amines lead to only limited cleavage of the phosphodiester bond of RNA as would be expected by a strong base.

To 1.45ml of the reaction containing the desired RNA analyte is added 1.4ml of ice cold 1-methylimidazole and the solution mixed by gentle pipetting. Then three separate aliquots of 200µl of a prepared solution of 1ml of 1-methylimidazole, 1ml of either ethanolamine or preferably ethylenediamine containing 50µl (40mg/ml) of phosphate treated hydroxylapatite Type I (Chemicell, Germany) are added and the reaction incubated for 2 minutes at 25°C, before the remaining 1.45ml of the 1-methylimidazole, primary amine and beads are added and mixed. The entire mixture is then slowly mixed using an end-over-end wheel for 10 minutes at 25°C to allow deprotection of the RNA and binding to the beads. However, agitating the mixture is not essential. The addition of the amine leads to an exothermic reaction, and the amine is therefore most easily added to the RNA containing reaction diluted into the buffer 1-methylimidazole. Alternatively, silica may be used in the place of hydroxylapatite to bind the nucleic acid.

Phosphate treatment of the beads is as follows ; to 1ml of hydroxylapatite Type I (50mg/ml) was added 2ml of 0.2M sodium phosphate (pH 7) and the beads are briefly mixed by inverting the tube. The beads are then collected with a magnet and the liquid discarded, the bead pellet is then resuspended in 2ml of water, briefly mixed, the beads collected and the water wash repeated once more, the beads are then resuspnded in 1ml of water. It has been found that hydroxylapatite beads treated in this way bind less protein and the RNA is more easily eluted than the non-phosphate treated beads.

The beads are then collected from the reaction using a magnetic stand (Dynal Norway) and the liquid discarded by pouring it away from the bead pellet. The beads are then resuspended in 1ml of 70% methanol/ethanol (1:1 vol/vol) and transferred to a fresh 2ml polypropylene centrifuge tube and collected using a magnetic stand (Promega, USA). The beads are washed two more times in 1ml of 70% methanol/ethanol and then washed three times in 100µl of water. It is important not to touch the beads with the pipette tip when they are in aqueous suspension because they tend to stick to plastic resulting in sample loss. The RNA is now ready for storage, transport or can be immediately eluted from the hydroxylapatite beads.

The nucleic acid can be released from the hydroxylapatite bead by a variety of means including the use of phosphate containing solutions or a divalent metal ion chelator such as EGTA. The mechanism of nucleic acid elution most probably involves a competition between the nucleic acid phosphate groups and the hydroxylapatite calcium atoms. It has been found that nucleotide triphosphate groups and metal ion chelators are particularly effective at displacing nucleic acids from the hydroxylapatite.

To the nucleic acid hydroxylapatite bead mixture (approximate volume 50-100µl) is added 50µl of a nucleotide solution, the beads agitated by means of a magnetic stirrer set on the program 0.5 second step, (MCB1200, Dexter Magnetics, UK) for 2 minutes at 25°C,the beads collected using the magnet, the liquid collected and then the process repeated 3 more times and the liquid containing the nucleic acid pooled. The nucleotide solution can be a 5mM dNTP solution such as dATP, dCTP, dGTP, TTP, dUTP or a 5mM solution of ribose NTP such as rATP, rCTP, rGTP, RUTS or a 50mM solution of dNDP or rNDP, DNMP or rNMP or even inorganic pyrophosphate (sodium phosphate) as described (Sambrook et al., (1989) Molecular Cloning: A Laboratory Manual, CSH). It has been found that as the nucleic acid is displaced from the hydroxylapatite, the total nucleotide concentration is reduced indicating it is binding to the hydroxylapatite in place of the eluted nucleic acid. This is important when calculating the final nucleotide concentration for downstream analytical procedures such as RT-PCR.

Alternatively, and preferably, the nucleic acid can be eluted from the hydroxylapatite using calcium ion chelators such as CDTA (trans-1,2-Diaminocyclohexane-N,N,N',N'-tetraacetic acid), EDTA (Etylenediamine tetraacetic acid), EGTA (Etylenenglycol-O,O'-bis(2-aminoethyl)-N,N,N',N'-tetraacetic acid), DTPA (Diethylenetriamine pentaacetic acid), HEDTA (N-(2-Hydorxyethyl)ethylenediamine-N,N,N'-triacetic acid), NTA (Nitrilotriacetic acid), TTHA (Triethylenetetramine-N,N,N',N",N"',N"'-hexaacetic acid), Dimethyl-BAPTA (Molecular Probes, USA) or BAPTA (Bis(2-aminophenoxy)ethane-N,N,N',N'-tetraacetic acid). To the nucleic acid hydroxylapatite bead mixture (approximate volume 50-100µl) is added 50µl of a 10mM chelator solution, the beads agitated by means of a magnetic stirrer set on the program 0.5 second step, (MCB1200, Dexter Magnetics, UK) for 2 minutes at 25°C, the beads collected using the magnet, the liquid collected and then the process repeated 7 more times and the liquid containing the nucleic acid pooled. It has been found that 75% of the nucleic acid is eluted between the 5th to the 8th elution (200-400µl) of a 10mM solution of EGTA. By comparison, it has also been found that DNA is eluted almost equally into the 1st to the 7th 50µl elution volume.e as determined by both 32P labelled DNA tracer studies and PCR amplification of the eluted DNA.

The eluted RNA and the material used to elute it from the hydroxylapatite can either be used directly in an analytical procedure such as hybridisation, or in an enzymatic assay such as reverse transcription-PCR. Whilst phosphate containing elution solutions work efficiently to elute nucleic acids from hydroxylapatite, they are not ideal because excess phosphate tends to interact and therefore reduce the final concentration of metal ions in enzymatic reactions leading to reduced yields. Therefore it is preferred to use metal ion chelators such as EGTA which have higher specificity for certain types of divalent metal ions. It has been found that when 10mM EGTA is used to elute the nucleic from the hydroxylapatite, the final EGTA concentration is approximately 4-5mM. EGTA has a lower stability constant (logK 5.21) for magnesium ions than EDTA (logK 8.69), therefore it has been found that the presence of at least 8mM (final EGTA concentration) in a RT-PCR reaction does not detectably alter the amplicon yield. Therefore for assays employing magnesium as a divalent metal cation at, for example 2mM or more, such as MULV, AMV and Taq DNA polymerases, EGTA is a good choice for eluting the nucleic acid. It is also possible to use a minimum amount of EGTA necessary to elute the RNA so that all the EGTA in solution is effectively captured by the magnetic-hydroxylapatite leaving the RNA essentially EGTA free as set out in example 29 However, it has also been found that EGTA/ nucleic acid solutions tend to chelate manganese ions such as those solutions employed in commercial diagnostic RT-PCR assays for example the Amplicor HCV Test, version 2.0 (Roche Diagnostics, USA) leading to reduced amplification yield when too much of the EGTA/ nucleic acid solution is added. It has been found for example that the addition of 6µl or more of the EGTA/ nucleic acid solution to a standard 100µl Amplicor HCV Test, version 2.0 leads to reduced OD660nm readings indicating that the amplification efficiency is reduced.

Where the assay employs manganese ions, there are several practical solutions to the problem of chelation and therefore inhibition of the amplification. Firstly the chelator can be removed using a differential binding process whereby the chelator molecule such as EGTA can diffuse through a semi-permeable material / barrier such as a gel, membrane, polymer or pore and bind to a chelator binding material such as hydroxylapatite. It has been found that suitable methods for removing small chelators from a nucleic acid solution are (i) mixing hydroxylapatite beads with 0.2-0.8% molten agarose or preferably polymerising acrylamide with hydroxylapatite and allowing it to solidify thereby entombing the hydroxylapatite. The EGTA/ nucleic acid solution is then placed in contact with the gel and the EGTA (or other chelator) allowed to diffuse into the gel, whilst the gel prevents the larger RNA molecules from contacting the beads and therefore from being lost. It has been found that 20 minutes is sufficient to remove 90% of the EGTA from a 50µl volume of 5mM EGTA solution at ambient temperature when placed in contact with a 1cm2 area of 100µl of the gel containing 0.5mg hydroxylapatite (type I, Chemicell, Germany). The remaining liquid containing the nucleic acid is then added to the assay. Alternatively, the hydroxylapatite beads can be coated in a polyner such as a heparin, cellulose, starch or dextran to provide a semi-permeable barrier to the nucleic acid whilst still allowing the chelator molecules to bind the hydroxylapatite. A 200µl volume of the polymer solution (10%) is conveniently added to 0.5mg hydroxylapatite (type I, Chemicell, Germany) beads and allowing the solution to dry completely at 90°C for 1 hour.

Another method (ii) for removing the chelator is to filter the nucleic acid / chelator solution. Although many filtration methods exist for selectively removing contaminants from nucleic acids, such as size exclusion chromatography and silica membranes, a preferred method is to use centrifugal or pressure filter devices, also known as ultrafiltration such as those know as Centricon, Centriprep, Centriplus and Centricon Plus® manufactured by Millipore (USA). In particular it has been found that Microcon centrifugal devices are well suited for this purpose. These devices use regenerated cellulose with various pore sizes with molecular weight cut offs from 3 000-100 000 daltons. The preferred size is 50 000 daltons molecular weight cut off (Part No. 42416, Millipore, USA) so that nucleic acids more than 200 nucleotides in length are retained by the membrane, whilst the chelator such as EGTA passes through the membrane and are discarded. The nucleic acid can be removed from the hydroxylapatite using a chelator, a phosphate containing solution, or a calcium binding salt. An advantage of the filtration method is that not only are contaminants removed but the nucleic acid is concentrated. This is particularly advantageous when the elution solution containing the nucleic acid is more than 100µl because the final filtered and concentrated volume can be 10µl so that the entire sample can be added to the analytical test. For example with the HCV Amplicor test v2.0 (Roche Diagnostics, USA) the maximum volume that may be added to each test is 50µl, whilst the eluted nucleic acid volume may be as much as 0.5ml. The filtration therefore serves two purposes ; to remove the contaminants and to concentrate the analyte.

The preferred method of filtration is as follows. Using 10mM EGTA as the elution solution, the first 400µl of the elution from the hydroxylapatite beads is collected and pooled. The elution can either be 8 separate elutions of 50µl with 2 minute elution steps using mixing as described above (MCB1200, Dexter Magnetics, USA) or 1 elution with 400µl of 10mM EGTA with a ten minute elution with mixing. It is also possible to add a smaller volume of more concentrated elution solution such as µl of 20mM EGTA and mixing for 10 minutes at room-temperature. In any case, the final elution volume is conveniently no more than 400 µl which is the maximum volume accomodated by the Microcon filtration device (Part No. 42416, Millipore, USA). The device is then centrifuged at 12 000g for 20 minutes (until dryness), 400µl of water added and the device spun again at 12 000g for 20 minutes (until dryness). Then 25-50µl of water is added to the device to recuperate the nucleic acid the cup inverted in a 2ml fresh tube and centrifuged for 10 seconds at 2500g. The nucleic acid can then be used in the assay. Proteins that are larger than 50 000 daltons are also retained with the nucleic acid, however, it has been found that no detectable inhibition occured during RT-PCR (HCV Amplicor v2.0, Roche Diagnostics, USA) with RNA prepared in this manner from blood plasma. Alternatively the nucleic acid can be eluted from the hydroxylapatite using 400µl of 100-500mM Sodium Phosphate (pH 7) and mixing for 10 minutes at room temperature. The phosphate/ nucleic acid solution can then be filtered using a Microcon filtration device as described above.

Yet another method (iii) for relieving the inhibitory effects of the chelator and in particular the EGTA solution is to 'neutralise' the capacity of the chelator to bind the manganese or magnesium. This can be accomplished in a number of ways. The first method is to add a metal ion that has a higher stability constant for the chelator than the metal ion necessary for the assay. Unfortunately Mn ions have a particularly high stability constant (12.3) with EGTA compared with Mg ions (5.21). Therefore it is preferable to choose metal ions with stability constants higher than Mn (12.3) so that the added metal ion competes effectively with the Mn for the chelator. Suitable metal ions are Fe(III) (20.3), Cu (II) (17.8), Co (II) (12.30) and Zn (II) (14.5). These metal ions can be added to the chelator containing solution as a salt such as the chloride or acetate. Although other metal ions also have high stability constants for EGTA, such as Ni, Hg and Cd, their toxicity would preclude their routine use. It has been found that adding 3.5-7mM final concentration of CoCl2 to a 100µl standard HCV Amplicor (Roche Diagnostics, USA) reaction containing 25µl of RNA eluted from the hydroxylapatite beads using 10mM EGTA effectively removes the inhibitory effects of the EGTA allowing amplification of the HCV analyte RNA. The most preferable CoCl2 concentrations are 4.5mM and 6mM. Alternatively, CuCl2 or FeCl3 can also be used in the range 3.5-7mM but are less effective.

It is also possible to use an additional amount of Mn in the analytical procedure to replace the Mn bound to the chelator. It has been found that adding 1-3mM, or preferably 1.5mM Manganese acetate to a 100µl standard HCV Amplicor reaction containing 25µl of RNA eluted from the hydroxylapatite beads using 10mM EGTA effectively removes the inhibitory effects of the EGTA allowing amplification of the HCV analyte RNA. Therefore it is not strictly necessary that the metal ion has a stability constant higher than Mn in order to overcome the inhibition. However, 4.5mM. CoCl2 is preferred in the HCV Amplicor test as described above.

RNA can also be eluted directly from the hydroxylapatite beads into a 5X solution of the RT-PCR buffer (250mM bicine/KOH buffer pH8.2, 575mM potassium acetate, 40% glycerol (w/v) used for Tth DNA polymerase and then added to the other reaction components (Roche Amplicor HCV v2.0) prior to amplification.

If silica particles or membranes are used for capturing the nucleic acid, then an equal weight of silica such as Qiaex II (Qiagen, Germany) or Magnisil (Promega, USA) is added in the place of hydroxylapatite beads. In this case the silica beads, following the deprotection reaction with the primary amine are washed in four rinses of 1ml of 70% ethanol and then the nucleic acid is eluted in 200µl of water following incubation for 10 minutes at 37°C.

### Example 2

### Purification of DNA from human blood plasma

Various types of DNA can be purified from plasma such as the clinically important virus HBV or bacteria. The viral or bacterial particles are disrupted in the presence of 1-methylimidazole, tetrahydrofuran (THF) and an acylating reagent such as acetic anhydride. This mixture also leads to the disruption of the nucleoprotein complex and consequent release of the DNA which can then be chemically modified and stabilised.

Methods for purifying DNA are substantially similar to methods for purifying RNA as set out in Example 1 above. However, unlike RNA, ds DNA (300bp) tends to elute from the first to the 7th addition of 50µl of 10mM EGTA, therefore all fractions of the elution should be kept.

### Example 3

### Purification of DNA and RNA simultaneously from human plasma

Both DNA and RNA can be purified from plasma at the same time, allowing simultaneous testing of both clinically important RNA sources such as HCV and HIV as well as DNA sources such as HBV from the same eluted nucleic acid sample. The viral or bacterial particles are disrupted in the presence of 1-methylimidazole, tetrahydrofuran (THF) and an acylating reagent such as acetic anhydride. This mixture also leads to the disruption of the nucleoprotein complex and consequent release of the DNA which can then be chemically modified and stabilised.

Methods for purifying DNA and RNA are substantially similar to methods set out in Example 1 and 2 above.

### Example 4

### Purification of DNA and/ or RNA from whole human blood

If present, both DNA and RNA can be purified from whole blood at the same time, allowing testing of both clinically important RNA sources such as HCV and HIV as well as DNA sources such as HBV from the same eluted nucleic acid sample.

Methods for purifying DNA and/ or RNA are substantially similar to Example 1 above, except that 200µl, instead of 50µl of 1-methylimidazole is added to 200µl of blood. It has been found that the addition of 200µl, instead of 50µl of 1-methylimidazole helps to solubilise the cellular components present in the blood as the erythrocytes and white blood cells. Although it has been found that 50µl of 1-methylimidazole also works well, the mixture of 1-methylimidazole, tetrahydrofuran and acetic anhydride tends to form large clumps which only dissolve once the deprotection reagent (e.g. ethylenediamine) is added.

Alternatively, 200µl of blood can be dissolved into 1ml of NMP :acetic anhydride (2 :1 vol :vol) and then 3ml of 1-methylimidazole added, followed by an incuabation of 10 min. at 37°C

### Example 5

### Purification of DNA and/ or RNA from cells

Both DNA and RNA can be purified from cells at the same time, allowing purification of both DNA and cellular RNA such as rRNA, tRNA and mRNA.

Methods for purifying DNA and/ or RNA are substantially similar to Example 1 above, except that 200µl, instead of 50µl of 1-methylimidazole is added to 200µl of a centrifuge collected pellet (3 000g x 10 minutes) of 1 million tissue culture cells. It has been found that the addition of 200µl, instead of 50µl of 1-methylimidazole helps to solubilise the cellular components present. Although it has been found that 50µl of 1-methylimidazole also works well, the mixture of 1-methylimidazole, tetrahydrofuran and acetic anhydride tends to form large clumps which only dissolve once the deprotection reagent (e.g. ethylenediamine) is added.

Alternatively, 50mg of tissue or organ sample can be disrupted using a dounce homegeniser or sonicator in the presence of 200µl of 1-methylimidazole, then the mixture immediately added to 600µl of THF/ acetic anhydride (2 :1 vol :vol), mixed before a second addition of 600µl of THF/ acetic anhydride (2 :1 vol :vol). Following a 10 minute incubation at 37°C the RNA is purified identically as for Example 1 starting at the addition of 1.4ml of 1-methylimidazole.

It has been found that a mixture of any one of the solvents N-methyl pyrrolidinone, Formyl morpholine or Dimethyl imidazolidone with acetic anhydride (2 :1 vol :vol) efficiently lyses only the cell membrane of tissue culture cells but not the nuclear membrane. Therefore cytoplasmic nucleic acids are preferrentially released and purified using these solvents. This is a useful method for reducing contamination with nuclear located genomic DNA.

### Example 6

### Purification of DNA from human faeces or urine

The method is as described as for example 1 except that the 200µl of plasma is replaced by 200µl of urine or 200µl of faeces diluted in water to 10%.

### Example 7

### Deprotection of acetyl modified RNA using gaseous ammonia

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded. The beads were washed once with 95% ethanol and allowed to air dry for 5 minutes at 25°C.

Deprotection of the modified RNA. The open tube containing the bead-modified RNA mixture was placed in a 50ml screw top polypropylene tube and a pipe inserted into a hole through the cap of the 50ml tube. 100ml of 38% ammonium hydroxide was heated to 50°C in a side arm flask, the ammonia vapour was allowed to exit the flask and enter a separate side arm glass flask to allow condensation of any water vapour. The dried ammoni gas was then allowed to enter the 50 ml tube containing the beads by means of a flexible plastic pipe. The screw cap of the 50ml tube was loosely closed allowing ammonia gas to enter the tube and then exit to be replaced by fresh ammonia.

The acetylated RNA on the beads was subjected to the ammonia for 5-60minutes, after which the beads were washed once in water and then eluted with 10mM EDTA or EGTA (hydroxylapatite) or the deprotected RNA was eluted directly into 50µl water (silica beads). It was found tht the time for deprotection varied according to the amount of ammonia produced during heating, 60 minutes usually being sufficient to remove substantially all acetyl groups from the RNA. The deprtotected RNA can then be used for various downstream applications such as RT-PCR and hybridisation.

### Example 8

### Deprotection of acetyl modified RNA using ethylenediamine

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil», Promega, USA) and mixed for 10 minutes at 25°C. The beads were then collected using a magnetic stand (Promega, USA) and the liquid discarded.

To the wet beads was added 50-500µl of ethylenediamine (Fluka cat No. 03550, France) and the beads stirred briefly and incubated for 1-60 minutes at 25°C. The beads were then collected with a magnet and the liquid discarded. The beads were washed twice with 200µl of 70% ethanol/methanol (1 :1) once with water and then the deprotected RNA was eluted into 100µl of 10mM EDTA or EGTA by incubating for 10 minutes at 25°C. It was found that 1, minute with 300µl of ethylenediamine was not sufficient to fully deprotect the RNA as assayed using an *in vitro* transcript labelled with 32P and a 5% acrylamide sequencing gel. The rate of migration is proportional to the amount of deprotection and sequencing gels serve as a useful measure of the amount of deprotection that has occured, After 5 minutes the RNA was over 90% deprotected and after 15 minutes the RNA was completely deprotected. Increasing the deprotection time to 60 minutes at 25°C did not lead to any detectable RNA degradation.

### Example 9

### Deprotection of acetyl modified RNA using ethanolamine

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin Germany) or magnetic silica particles (« Magnisil », Promega, USA) and mixed for 10 minutes at 25°C. The beads were then collected using a magnetic stand (Promega, USA) and the liquid discarded.

To the beads that were not dried from the previous protection reaction, was added 50-500µl of ethanolamine (Fluka cat. No. 02400, France) and the beads stirred briefly and incubated for 1-60 minutes at 25°C. The beads were then collected with a magnet and the liquid discarded. The hydroxylapatite beads were washed twice with 200µl of 70% ethanol/methanol (1 :1), once with water and then the deprotected RNA was eluted into 100µl of 10mM EDTA or EGTA by incubating for 10 minutes at 25°C. The silica beads were washed twice in 200µl of Wash solution PE (Qiagen, Germany) and the deprotected RNA eluted in 100µl of water.

It was found that 1 minute with 300µl of ethylenediamine was not sufficient to fully deprotect the RNA as assayed using an *in vitro* transcript labelled with 32P and a 5% acrylamide sequencing gel. The rate of migration is proportional to the amount of deprotection and sequencing gels serve as a useful measure of the amount of deprotection that has occured. After 5 minutes the RNA was over 70% deprotected and after 15 minutes the RNA was was over 90% deprotected. Complete deprotection occured after 30 minutes at 25°C. Increasing the deprotection time to 60 minutes at 25°C led to detectable degradation of a 1700 nucleotide RNA molecule but relatively little degradation of a 250 nucleotide molecule as determined by sequencing gel analysis. Therefore deprotection with ethanolamine was slightly slower than with ethylelenediamine and also led to more RNA degradation. However, this may have been at least in part due to the purity of the ethanolamine.

### Example 10

### Deprotection of acetyl modified RNA using mixtures of ethanolamine and ethylenediamine

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded.

To the wet beads was added 50-500µl of a mixture of ethanolamine and ethylenediamine (1 :1), (Fluka, France) and the beads stirred briefly and incubated for 1-60 minutes at 25°C. The beads were then collected with a magnet and the liquid discarded. The hydroxylapatite beads were washed twice with 200µl of 70% ethanol/methanol (1 :1) once with water and then the deprotected RNA was eluted into 100µl of 10mM EDTA or EGTA by incubating for 10 minutes at 25°C. The silica beads were washed twice in 200µl of Wash solution PE (Qiagen, Germany) and the deprotected RNA eluted in 100µl of water.

### Example 11

### Deprotection of acetyl modified RNA using ethanolamine or ethylenediamine at increased temperatures

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded.

To the wet beads was added 100µl of ethylenediamine or ethanolamine and the beads stirred briefly and incubated for 20 minutes at 37,45 and 55°C. The beads were then collected with a magnet and the liquid discarded. The beads were washed twice with 200µl of 70% ethanol/methanol (1 :1) once with water and then the deprotected RNA was eluted into 100µl of 10mM EDTA or EGTA by incubating for 10 minutes at 25°C.

The amount of deprotection of the RNA as assayed using an *in vitro* transcript labelled with 32P and a 5% acrylamide sequencing gel. The rate of migration is proportional to the amount of deprotection and sequencing gels serve as a useful measure of the amount of deprotection that has occured. It was found that with ethanolamine at 55, 45 or 37°C there was significant degradation of the RNA, whilst with ethylenediamine at 55°C there was limited degradation, both deprotection at 45 or 37°C did not lead to detectable RNA degradation. Thgerefore deprotection with ethanolamine is best achived at 25°C whilst with ethylenediamine, deprotection can be carried out up to 45°C.

### Example 12

### Deprotection of acetyl modified RNA using ethanolamine or ethylenediamine in alcohol

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded.

To the wet beads was added 100µl of ethylenediamine or ethanolamine and 100µl of methanol/ethanol (1:1) and the beads stirred briefly and incubated for 20 minutes at 37, 45 and 55°C. The beads were then collected with a magnet and the liquid discarded. The beads were washed twice with 200µl of 70% ethanol/methanol (1:1) once with water and then the deprotected RNA was eluted into 100µl of 10mM EDTA or EGTA by incubating for 10 minutes at 25°C.

It was found that the addition of alcohol was not beneficial to the deprotection reaction, indeed the rate of deprotection was reduced whilst the amount of RNA degradation was increased.

### Example 13

### Deprotection of acetyl modified RNA using ethanolamine or ethylenediamine with a strong alkali

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded.

To the wet beads was added 100µl of ethanolamine plus either 10µl of 10% ammonium hydroxide, 10µl of 10mM NaOH or 10µl of 50mM NaOH and the beads stirred briefly and incubated for 20 minutes at 37°C. The beads were then collected with a magnet and the liquid discarded. The beads were washed twice with 200µl of 70% ethanol/methanol (1 :1) once with water and then the deprotected RNA was eluted into 100µl of 10mM EDTA or EGTA by incubating for 10 minutes at 25°C.

It was found that the addition of either ammonium hydroxide or NaOH did not increase the amount of deprotection compared with ethanolamine alone bu did increase the amount of RNA degradation.

### Example 14

### Deprotection of acetyl modified RNA using ethanolamine and ethylenediamine in the presence of water

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded.

To the wet beads was added 50µl of ethanolamine, 10µl of water and 50µl of methanol and the beads stirred briefly and incubated for 5 minutes at 25°C. The beads were then collected with a magnet and the liquid discarded. The beads were washed twice with 200µl of 70% ethanol/methanol (1 :1) once with water and then the deprotected RNA was eluted into 100µl of 10mM EDTA or EGTA by incubating for 10 minutes at 25°C.

It was found that the addition of water did not alter the amount of deprotection compared with ethanolamine and alcohol alone and did not increase the amount of RNA degradation. It is therefore not essential that water be removed from solutions and the beads prior to deprotection.

### Example 15

### Alternative means to remove RNA from hydroxylapatite beads

It has been found that either protected or deprotected RNA can be removed from hydroxylapatite beads by simply loading the bead-RNA complex into either a well of a 0.5 X TAE agarose gel or a well of a 1 X TBE sequencing gel and applying an electric field through the well containing the beads. The protected or deprotected RNA readily dissociates from the hydroxylapatite beads and electrophoresis into the gel where it can be either collected or analysed by means for example of EtBr or a radioactive label. This is a very convenient means to detach RNA from hydroxylapatite.

Protected or deprotected RNA may also be separated from hydroxylapatite by inserting 2 wires (anode and cathode) without them touching into a tube containing beads in 100 1 of water or buffer and applying a low voltage such as 5-50V for 5 minutes. The RNA can be recuperated from the liquid phase.

### Example 16

### RT-PCR amplification of Deprotected RNA

Preparation of modified template RNA. To 100µl of a mixture of tetrahydrofuran/acetic anhydride (2 :1 vol/vol) was added 14µl of 1-methylimidazole and 2µg of BMV RNA (Promega, USA), the mixture stirred and incubated for 2 minutes at 25°C. To the reaction was added 60µl of 1-butanol and then 20µl of magnetic hydroxylapatite Type 1 (40mg/ml) (Chemicell GmbH, Berlin, Germany) and mixed for 3 minutes at 25°C. The beads were then collected using a magnetic stand (Promega, USA), washed once in 200µl of 70% methanol/ ethanol (1:1) and the liquid discarded.

To the wet beads was added either 100µl of ethanolamine or 100µl of ethylenediamine and the beads stirred briefly and incubated for 20 minutes at 37°C. The beads were then collected with a magnet and the liquid discarded. The beads were washed twice with 200g of 70% ethanol/methanol (1 :1) once with water and then the deprotected RNA was eluted into 100µl of 10mM EGTA by incubating for 10 minutes at 25°C.

Reverse Transcription. 25 ng of the deprotected BMV RNA was added to a 20 µl reaction mixture containing the following final component concentrations: 200 mM Tris-HCl (pH 8.4 at 24°C), 75 mM KCl, 2.5 mM MgCl₂, 10 mM DTT, 1 mM dNTP's, 60 ng of oligonucleotide primer BMV R (GAGCCCCAGCGCACTCGGTC) and MULV RNase H⁻ (Promega, cat no. M3682, USA). Water was used to bring the final volume to 20 µl. The reaction was allowed to proceed for 20 minutes at 37°C, 20 minutes at 42°C and 20 minutes at 50°C. PCR Amplification. The PCR was carried out in a final volume of 25µl with final concentration of 15mM Tris-HCl pH 8.8, 60mM KCl, 2.5mM MgCl₂, 400 µM each dNTP, 10 pmol of each primer BMV F (CTATCACCAAGATGTCTTCG) and BMV R and 1 unit *Taq* DNA polymerase (Roche Molecular, France). To the PCR mix was added 2 µl of cDNA generated from the deprotected BMV RNA. Cycle parameters were 94°C x 8 sec, 58°C x 8 sec and 72°C x 15 sec for 30 cycles. The 250bp PCR products were visualised following gel electrophoresis and staining with EtBr.

Excellent amplification was observed with both ethylenediamine and ethanolamine deprotected RNA, indeed no significant differences could be seen in the yield of PCR product between protected-deprotected RNA compared with an untreated RNA control indicating that no substantial degradation of the RNA occured during deprotection.

### Example 17

### Hybridisation of Deprotected RNA

Preparation of modified template RNA. To 100µl of a mixture of tetrahydrofuran/acetic anhydride (2:1 vol/vol) was added 14µl of 1-methylimidazole and 2µg of BMV RNA (Promega, USA), the mixture stirred and incubated for 2 minutes at 25°C. To the reaction was added 60µl of 1-butanol and then 20µl of magnetic hydroxylapatite Type 1 (40mg/ml) (Chemicell GmbH, Berlin, Germany) and mixed for 3 minutes at 25°C. The beads were then collected using a magnetic stand (Promega, USA), washed once in 200µl of 70% methanol/ ethanol (1:1) and the liquid discarded.

To the wet beads was added either 100µl of ethanolamine or 100µl of ethylenediamine and the beads stirred briefly and incubated for 20 minutes at 37°C. The beads were then collected with a magnet and the liquid discarded. The beads were washed twice with 200µl of 70% ethanol/methanol (1:1) once with water and then the deprotected RNA was eluted into 100µl of 10mM EGTA by incubating for 10 minutes at 25°C.

Immobilisation of deprotected RNA. 100, 50 or 25 ng of the deprotected BMV RNA was added to a 20 µl reaction mixture containing the following final component concentrations: 200 mM Tris-HCl (pH 8.4 at 24°C), 75 mM KCl, 2.5 mM MgCl₂, 10 mM DTT, 1 mM dNTP's, 60 ng of oligonucleotide primer BMV R (GAGCCCCAGCGCACTCGGTC) and MULV RNase H⁻ (Promega, cat no. M3682, USA). Water was used to bring the final volume to 20 µl. The reaction was allowed to proceed for 20 minutes at 37°C, 20 minutes at 42°C and 20 minutes at 50°C. PCR Amplification. The PCR was carried out in a final volume of 25µl with final concentration of 15mM Tris-HCl pH 8.8,60mM KCl, 2.5mM MgCl₂, 400 M each dNTP, 10 pmol of each primer BMV F (CTATCACCAAGATGTCTTCG) and BMV R and 1 unit *Taq* DNA polymerase (Roche Molecular, France). To the PCR mix was added 2 µl of cDNA generated from the deprotected BMV RNA. Cycle parameters were 94°C x 8 sec, 58°C x 8 sec and 72°C x 15 sec for 30 cycles. The 250bp PCR products were visualised following gel electrophoresis and staining with EtBr.

Excellent amplification was observed with both ethylenediamine and ethanolamine deprotected RNA, indeed no significant differences could be seen in the yield of PCR product between protected-deprotected RNA compared with an untreated RNA control indicating that no substantial degradation of the RNA occured during deprotection.

### Example 18

### Deprotection of propanoyl modified RNA using ethylenediamine

Preparation of modified RNA. To 40µl of tetrahydrofuran containing 16% 1-methylimidazole was added 100ng-1µg of RNA, then 2-4µl of propionic anhydride (Fluka cat. No. 81942) was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles («Magnisil », Promega, USA) and mixed for 10 minutes at 25°C. The beads were then collected using a magnetic stand (Promega, USA) and the liquid discarded.

To the wet beads was added 50-500µl of ethylenediamine (Fluka cat. No. 03550, France) and the beads stirred briefly and incubated for 1-60 minutes at 25°C. The beads were then collected with a magnet and the liquid discarded. The beads were washed twice with 200µl of 70% ethanol/methanol (1:1) once with water and then the deprotected RNA was eluted into 100µl of 10mM EDTA or EGTA by incubating for 10 minutes at 25°C. It was found that 1 minute with 300µl of ethylenediamine was not sufficient to fully deprotect the RNA as assayed using an *in vitro* transcript labelled with 32P and a 5% acrylamide sequencing gel. The rate of migration is proportional to the amount of deprotection and sequencing gels serve as a useful measure of the amount of deprotection that has occured. After 5 minutes the RNA was over 90% deprotected and after 15 minutes the RNA was completely deprotected. Increasing the deprotection time to 60 minutes at 25°C did not lead to any detectable RNA degradation.

### Example 19

### Deprotection of acetyl modified RNA using polymer bound ethylenediamine

Ethylenediamine and several other types of primary amines are commercially available bound to a solid (polymer) support. Such primary amines are suitable for deprotecting acetylated RNA. They provide a convenient method for removing the deprotection reagent from the reaction once the deprotection is complete.

Preparation of modified RNA. To 40µl of tetrahydrofuran containing 16% 1-methylimidazole was added 100ng-1µg of RNA, then 2-4µl of acetic anhydride (Fluka cat. No. 81942) was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil » , Promega, USA) and mixed for 10 minutes at 25°C. The beads were then collected using a magnetic stand (Promega, USA) and the liquid discarded. The acetylated RNA on the hydroxylapatite beads were eluted into 200µl of 10mM EDTA or EGTA by incubating for 10 minutes at 25°C. The chelator/acetylated RNA solution (200µl) was then added to 100mg of ethylenediamine beads (Sigma-Aldrich-Alrdich Part No. 54,748,4, USA) and incubated for 1hr at 37°C. The beads are conveniently removed by centrifugation (15 000rpm for 10 seconds) or filtration (Microcon device, Millipore, USA) leaving the deprotected RNA in solution.

### Example 20

### Deprotection of acetyl modified RNA using Propylenediamine

Preparation of modified RNA. To 40µl of tetrahydrofuran containing 16% 1-methylimidazole was added 100ng-1µg of RNA, then 2-4µl of acetic anhydride was mixed into the reaction and incubated for 10 minutes at 37°C. To the reaction was added 10µl of magnetic hydroxylapatite Type 1 (40mg/ml) (Chemicell GmbH, Berlin, Germany) or magnetic silica particles (« Magnisil », Promega, USA) and mixed for 10 minutes at 25°C. The beads were then collected using a magnetic stand (Promega, USA) and the liquid discarded.

To the beads that were not dried from the previos protection reaction, was added 50-500µl of propylenediamine (Fluka cat. No. 82250, France) and the beads stirred briefly and incubated for 1-60 minutes at 25-37°C. The beads were then collected with a magnet and the liquid discarded. The hydroxylapatite beads were washed twice with 200µl of 70% ethanol/methanol (1 :1), once with water and then the deprotected RNA was eluted into 100µl of 10mM EDTA or EGTA by incubating for 10 minutes at 25°C. The silica beads were washed twice in 200µl of Wash solution PE (Qiagen, Germany) and the deprotected RNA eluted in 100µl of water.

### Example 21

### Deprotection of acetyl modified RNA using diethylenetriamine or tetraethylenetriamine

To 200µl of human plasma or serum containing the nucleic acid to be stabilised and purified was added 50µl of 1-methylimidazole and briefly mixed. To this mixture was added 1ml of N-methyl pyrrolidinone/ acetic anhydride (2 :1 vol :vol), mixed briefly and incubated for 10 minutes at 26-37°C. To the mixture was then added 1ml of 1-methylimidazole, mixed and incubated for 2 minutes at 26°C and then 1.6ml of 1-methylimidazole/ diethylenetriamine (Fluka, USA) (1 :1 vol :vol) containing 500µl of magnetic-hydroxylapatite beads (Chemicell, Germany). The mixture was gently inverted by rotation (LabQuake, USA) for 10 minutes before the magnetic-hydroxylapatite beads were collected using a permanent magnet (Promega, USA). The liquid was discarded and the beads washed with 1ml of 70% ethanol, collected with a magnet and washed with 200µl of water, collected with a magnet, the liquid discarded and then to the beads was added 200µl of 10mM EGTA pH 9.9 (NH4OH salt) elution solution and mixed for 4 minutes using a magnetic mixer (Dexter Magnetics, UK). The beads were collected and the liquid containing the RNA transferred to a fresh tube, then a second batch of 200µl of 10mM EGTA pH 9.9 (NH4OH salt) elution solution was added to the beads, and following 4 minutes mixing and bead collection, the liquid pooled with the first elution and the RNA concentrated using a Microcon ultracentrifugation device as set out in example 1.

Alternatively, triethylenetetramine can be substituted for diethylenetriamine in the deprotection reaction. The advantage of triethylenetetramine is that it is less volataile and reactive compared with diethylenetriamine, however reaction times for complete deprotection may be slightly slower with the larger amine. Generally, amine bearing molecules which undergo substantial hydrogen bonding between molecules or have a larger molecular weight are less volatile and therefore preferred.

Alternatively, propionic (Fluka, USA), acrylic (Roth-Sochiel, Germany) or butyric anhydrides (Fluka, USA) can be subsituted for acetic anhydride in this example.

### Example 23

### Deprotection of acetyl modified RNA using Lysine or Arginine aqueous solutions

To 200µl of human plasma or serum containing the nucleic acid to be stabilised and purified was added 50µl of 1-methylimidazole and briefly mixed. To this mixture was added 1ml of N-methyl pyrrolidinone/ acetic anhydride (2 :1 vol :vol), mixed briefly and incubated for 10 minutes at 26-37°C. To the mixture was added 30µl of a solution of 3.45M lysine, mixed and incubated for 2 min. at 26°C, then 50µl of magnetic-hydroxylapatite Type I (Chemicell, Germany) was added and mixed for 5 min. at 26°C (MCB 1200, Dexter Magnetics, UK) to capture the nucleic acid. The magnetic-hydroxylapatite beads were collected with a magnet (Promega, USA) and briefly washed in 0.6ml of 70% ethanol, before the beads were once again collected by magnet, the wash liquid discarded and 0.5ml of a 3.45M Lysine solution or alternatively, 1ml of a 1M Arginine solution was added and mixed for 10 minutes at 26°C (MCB 1200, Dexter Magnetics, UK), the beads collected with a magnet and the liquid discarded. The beads were then washed with 0.6ml of 70% ethanol, the beads collected and the wash discarded and then washed with 0.2ml of water, the beads collected and the wash discarded followed by elution of the deprotected RNA using 0.2ml of a 10mM TEAH-EGTA solution pH 10.1 with mixing for 5 min. (MCB 1200, Dexter Magnetics, UK). The eluted RNA can be separated from the remaining EGTA by for example ultracentrifugation using a Microcon device as set out in example 1.

### Example 24

### Elution of RNA from hydroxylapatite using ammonium salts of EGTA

It has been found that ammonium salts of EGTA and other chelators are more effective at eluting nucleic acids from hydroxylapatite than sodium or potassium salts. A 10mM solution of EGTA was prepared by adding, dropwise, a 38% solution of NH4OH to a mixture of EGTA in water until the pH was brought to 9.9. This EGTA solution can be used as described in example 22 as an elution solution.

### Example 25

### Elution of RNA from hydroxylapatite using tetra-alkyl ammonium salts of EGTA

It has been found that tetralkylammonium salts of EGTA and other chelators are more effective at eluting nucleic acids from hydroxylapatite than ammonium (NH3) salts. A 10mM solution of EGTA was prepared by adding, dropwise, a solution of either tetramethylammonium, tetraethylammonium or tetrabutylammonium hydroxide to a mixture of EGTA in water until the pH was brought to 9.9. It was found that tetraethylammonium-EGTA was marginally the most effective of the three types. This EGTA solution can be used as an alternative to the 10mM EGTA pH 9.9 (NH4OH salt) elution solution as described in example 22.

### Example 26

### Deprotection of acetyl modified RNA using amine containing dendrimers

To 20µl of water containing 5ng of acetylated BMV RNA was added 1mg of a phosphodendrimer-NH3 (Loup et al., (1999) Chem. Eur. J. 5:3644) and incubated for 13 minutes at 37°C. The dendrimer was removed by centrifugation at 13 000g for 30 seconds and the deprotected RNA analysed. It was found that the dendrimer effectively removed the acetyl groups from the RNA.

### Example 26

### Removal of chelating activity using photosensitive chelators

RNA was isolated essentially as described in example 22 except the 10mM EGTA pH 9.9 (NH4OH salt) elution solution was replaced with 10mM DM-nitrophen (Calbiochem, USA) pH 8.0 elution solution. The elution solution containing the nucleic acid and the excess unwanted DM-nitrophen was subjected to photolysis essentially as described (Kaplan et al., (1985) Proc. Natl. Acad. Sci. 85 :6571) in order to remove the chelating activity of the DM-nitrophen thereby leaving the chelator free RNA ready to be used in an appropriate reverse transcription-PCR assay.

### Example 27

### Reduced reagent volumes

The ability to acetylate RNA using reduced volumes of solvent and acetic anhydride was examined. Acetylation was tested by the ability to modify a a 32P labelled RNA transcript, the percentage modification was estimated using the altered mobility of the transcript in a denaturing sequencing gel as set out in WO/00/75302. To 200µl of human plasma or serum containing the nucleic acid to be stabilised and purified was added 50µl of 1-methylimidazole and briefly mixed. To this mixture was added 0.4ml, 0.6ml, 0.8ml, 1ml or. 1.2ml of dioxolane/ acetic anhydride (2 :1 vol :vol), mixed briefly, then 5µl of 32P labelled RNA transcript (Riboprobe, Promega, USA) and incubated for 10 minutes at 37°C. The 32P labelled RNA was then recovered from the reaction by adding 50µl of Type I hydroxylapatite beads (Chemicell, Germany), incubating 5 min. with mixing, washing with 1ml of 70% ethanol and eluting using 10mM EGTA pH9.9. The RNA was then loaded on a sequencing gel to determine the extent of modification. It was found that the extent of RNA modification was proportional to the volume of reagent added, except that essentially the addition of 1ml or 1.2ml dioxolane / acetic anhydride (2 :1) mixture led to the same amount of modification. Therefore the minimum amount of dioxolane / acetic anhydride (2 :1) mixture necessary for maximum acetylation was determined to be between 0.8-1ml. It was found that using 0.6ml of dioxolane / acetic anhydride (2 :1) mixture that there was a small increase in the amount of acetylation between samples incubated at 37°C for 1, 10 or 30 min. The amount of acetylation also varied slightly according to the type of biological sample containing the RNA; RNA mixtures with human serum (Sigma-Aldrich, USA) being slightly less acetylated than human plasma or fetal calf serum.

### Example 28

### Deprotection of acetyl modified RNA following removal of the reaction

To 200µl of human plasma or serum containing the nucleic acid to be stabilised and purified was added 50µl of 1-methylimidazole and briefly mixed. To this mixture was added 1ml of N-methyl pyrrolidinone/ acetic anhydride (2 :1 vol :vol), mixed briefly and incubated for 10 minutes at 26-37°C. To the mixture was added 30µl of a solution of 3.45M lysine, mixed and incubated for 2 min. at 26°C, then 50µl of magnetic-hydroxylapatite Type I (Chemicell, Germany) was added and mixed for 5 min. at 26°C (MCB 1200, Dexter Magnetics, UK) to capture the nucleic acid. The magnetic-hydroxylapatite beads were collected with a magnet (Promega, USA) and briefly washed in 0.6ml of 70% ethanol, before the beads were once again collected by magnet, the wash liquid discarded and 10-500µl of diethylenetriamine was added to the wet beads and mixed for 10 minutes at 26°C (MCB 1200, Dexter Magnetics, UK), the beads collected with a magnet and the liquid discarded. The beads were then washed with 0.6ml of 70% ethanol, the beads collected and the wash discarded and then washed with 0.2ml of water, the beads collected and the wash discarded followed by elution of the deprotected RNA using 0.2ml of a 10mM TEAH-EGTA solution pH 10.1 with mixing for 5 min. (MCB 1200, Dexter Magnetics, UK). The eluted RNA can be separated from the remaining EGTA by, for example ultracentrifugation using a Microcon device as set out in example 1.

### Example 29

### Limiting amounts of elution solution

It has been found that replacing the elution solution as set out in example 22, with a single elution of 100µl of 10mM EGTA (NH4OH salt) pH 9.9 and an extended elution time of 30 minutes at 26°C with mixing (MCB 1200, Dexter Magnetics, UK) is sufficient to remove a significant proportion of the RNA from the magnetic-hydroxylapatite without significantly contaminating the eluted RNA with EGTA, so the RNA can be used directly without further removal steps of the EGTA. Effectively all the EGTA added is effectively bound to the magnetic-hydroxylapatite.

### Example 30

### Stability of mixtures of reagents

The stability of a mixture of anhydrides with various solvents and catalysts was examined. The activity of the acetic anhydride after storage for 11 days at 26°C was tested by the ability to modify a a 32P labelled RNA transcript, the percentage modification was estimated using the altered mobility of the transcript in a denaturing sequencing gel as set out in WO/00/75302. It was found that the following mixtures were active after storage for 11 days at 26°C ; N-methyl pyrrolidinone/ acetic anhydride/ 1-methylimidazole (2:1 :0.15, vol :vol :vol), N-methyl pyrrolidinone/ propionic anhydride/ 1-methylimidazole (2 :1 :0.15, vol :vol :vol), N-methyl pyrrolidinone/ acetic anhydride/ 4-pyrrolidinopyridine (2 :1 :0.15, vol :vol :vol). Intermediate activity was determined for the mixture N-methyl pyrrolidinone/ propionic anhydride/ 4-pyrrolidinopyridine (2 :1 :0.15, vol :vol :vol), and essentially inactive mixtures were N-methyl pyrrolidinone/ acetic anhydride/ 2-hydroxypyridine (2:1 :0.15, vol :vol :vol) and N-methyl pyrrolidinone/ acetic anhydride/ 2-hydroxypyridine (2:1 :0.15, vol :vol :vol). In these examples, the solvent N-methyl pyrrolidinone can be replaced by either formyl morpholine or dimethyl imidazolidone. Mixtures of solvent, acetic anhydride and 1-methylimidazole turned from a clear to a dark brown colour after 1 hr at room temperature, whilst this colour change was less apparent in propionic anhydride containing mixtures.

### Example 31

### Reactions using other catalysts

To 200µl of human plasma or serum containing the nucleic acid to be stabilised and purified was dissolved 50mg of either 4-pyrrolidinopyridine or 2-hydroxypyridine catalysts instead of 1-methylimidazole. To this mixture was added 1ml of N-methyl pyrrolidinone/ acetic anhydride (2 :1 vol :vol), mixed briefly and incubated for 10 minutes at 26-37°C. The RNA was then deprotected and purified according to the method of example 22. It was found that either 4-pyrrolidinopyridine or 2-hydroxypyridine catalysts could substitute for 1-methylimidazole.

Alternatively, propionic (Fluka, USA), acrylic (Roth-Sochiel, Germany) or butyric anhydrides (Fluka, USA) can be substituted for acetic anhydride in this example.

### Example 31

### Protection of modified RNA from freeze-thaw degradation

Preparation of modified RNA. To 40µl of tetrahydrofuran containing 16% 1-methylimidazole was added 100ng-1µg of BMV RNA (Promega, USA), then 2-4µl of acetic anhydride was mixed into the reaction and incubated for 10 minutes at 37°C. Following ethanol precipitation of the modified RNA and resuspension in 40µl of water, an aliquot of both modified and non-modified BMV RNA was put in separate 2ml polypropylene tubes and frozen in a mixture of dry-ice ethanol for 20 seconds followed by thawing at 42°C for 30 seconds. This cycle was repeated ten times in total. The RNA was mixed with 50% formamide and loaded on a 1.2% agarose gel. Whilst the unmodified RNA was significantly degraded, the modified RNA showed no signs of degradation by freeze thawing.

### Example 32

### Extraction of HCV viral RNA from clinical samples ; 1.45ml reaction volume

Stabilisation of the RNA analyte: To 200µl of human plasma (EDTA coagulation inhibitor) or serum in a 15ml screw top polypropylene centrifuge tube (Falcon, USA) was added 50µl of 1-methylimidazole, the mixture mixed briefly and then 600µl of a mixture of tetrahydrofuran and acetic anhydride (2 :1 vol/vol) was added and mixed by gentle pipetting with a 1ml pipette tip. A second addition of 600µl of tetrahydrofuran and acetic anhydride (2 :1 vol/vol) was added within 1 minute of the first addition and the solution mixed again.

Following addition of the acylation reagent, the internal control (8µl of the HCV Internal Control, version 2.0, Roche Diagnostics, USA) is added within 15 seconds. Following an incubation of 10 minutes at 37°C, the solution containing the stabilised RNA can be stored for prolonged periods at up to 37°C.

To 1.45ml of the reaction containing the desired RNA analyte is added 1.4ml of ice cold 1-methylimidazole and the solution mixed by gentle pipetting. Then three separate aliquots of 200µl of a prepared solution of 1ml of 1-methylimidazole, 1ml of ethylenediamine containing 50µl (40mg/ml) of phosphate treated hydroxylapatite Type I (Chemicell, Germany) are added and the reaction incubated for 2 minutes at 25°C, before the remaining 1.45ml of the 1-methylimidazole, primary amine and beads are added and mixed. The entire mixture is then slowly mixed using an end-over-end wheel for 10 minutes at 25°C to allow deprotection of the RNA and binding to the beads. However, agitating the mixture is not essential.

The beads are then collected from the reaction using a magnetic stand (Dynal, Norway) and the liquid discarded by pouring it away from the bead pellet. The beads are then resuspended in 1ml of 70% methanol/ethanol (1 :1 vol/vol) and transferred to a fresh 2ml polypropylene centrifuge tube and collected using a magnetic stand (Promega, USA). The beads are washed two more times in 1ml of 70% methanol/ethanol and then washed three times in 100µl of water. It is important not to touch the beads with the pipette tip when they are in aqueous suspension because they tend to stick to plastic resulting in sample loss. The RNA is now ready for storage, transport or can be immediately eluted from the hydroxylapatite beads.

To the nucleic acid hydroxylapatite bead mixture (approximate volume 50-100µl) is added 50µl of a 10mM EGTA (NaOH buffered) solution (pH 8), the beads agitated by means of a magnetic stirrer set on the program 0.5 second step, (MCB1200, Dexter Magnetics, UK) for 2 minutes at 25°C, the beads collected using the magnet, the liquid collected and then the process repeated 7 more times and the liquid containing the nucleic acid pooled.

The preferred method of filtration for concentration and removal of EGTA is as follows. Using 10mM EGTA as the elution solution, the first 400µl of the elution from the hydroxylapatite beads is collected and pooled. The elution can either be 8 separate elutions of 50µl with 2 minute elution steps using mixing as described above (MCB1200, Dexter Magnetics, USA) or 1 elution with 400µl of 10mM EGTA with a ten minute elution with mixing. It is also possible to add a smaller volume of more concentrated elution solution such as µl of 20mM EGTA and mixing for 10 minutes at room-temperature. In any case, the final elution volume is conveniently no more than 400 µl which is the maximum volume accomodated by the Microcon filtration device (Part No. 42416, Millipore, USA). The device is then centrifuged at 12 000g for 20 minutes (until dryness), 400µl of water added and the device spun again at 12 000g for 20 minutes (until dryness). Then 25-50µl of water is added to the device to recuperate the nucleic acid the cup inverted in a 2ml fresh tube and centrifuged for 10 seconds at 2500g. The nucleic acid can then be used in the assay. Proteins that are larger than 50 000 daltons are also retained with the nucleic acid, however, it has been found that no detectable inhibition occured during RT-PCR (HCV Amplicor v2.0, Roche Diagnostics, USA) with RNA prepared in this manner from blood plasma.

It has been found that HCV viral RNA can be detected at 300 copies per ml (as determined by Roche Amplicor Monitor version 1.0) of plasma using this stabilisation and purification method (see table 2).

**Table 2. Summary of results obtained using MRT (method as described in example 32) compared with Roche purification (Amplicor v2.0). S /CO represents the value of the OD 660nm reading divided by the OD 660nm cut-off value of 0.15.**

| **Identification** | **Génotype** | **Copies/ml *(Amp licor Monitor HCV Version1 .0)*** | **Particularités** | **MRT Purification** | **Roche Purification** | **MRT Purification *Incubation 37°C**** | **Roche Purification *Incubation 37°C**** |
|---|---|---|---|---|---|---|---|
| | | | | **S/CO** | **S/CO** | **S/CO** | **S/CO** |
| ETS.**2** | 1a | 2 500 | | ≥ 26.67 | 24.60 | 0.41 | 0.04 |
| ETS.**4** | 2a/2c | 300 | | 16.27 | 8.00 | 0.71 | 0.033 |
| ETS.**7** | 1 | 40 000 000 | | 25.8 | 25.73 | ≥ 26.67 | ≥ 26.67 |
| ETS.**10** | 3a | < seuil | | 2.13 | 1.67-9.33 -1933 | 0.033 | 0.04 |
| ETS.**12** | 5a | 500 | | ≥26.67 | 10.2 | 0.027 | 0.04 |
| ETS.**21** | 1b | 306 800 | | 24.64 | 17.27 | ≥ 26.67 | 25.81 |
| ETS.**25** | 5a | 3 300 | | ≥ 26.67 | 22.67 | 16.21 | 0.046 |
| ETS.**26** | 1a | 422 700 | Anti HCV (-) | 25.81 | 24.66 | 25.8 | 25.81 |
| ETS.**32** | 3a | 69 500 | +HBV | ≥ 26.67 | 25.8 | 8.63 | 0.046 |
| ETS.**34** | 1b | 364 000 | +HGV | 25.81 | 24.67 | 22.63 | 10.65 |
| Rim control | 1b | / | 50IU/ml | 21.15 | / | 0.8 | / |

### Example 33

### Extraction of HCV viral RNA from clinical samples following an incubation at 37°C for one week

To test the stabilising activity of modifying RNA, HCV positive plasma samples were mixed with the reagents as set out in example 32, but prior to the addition of 1.4ml of ice cold 1-methylimidazole, the samples were incubated at 37°C for one week. Following the incubation, the purification of the HCV RNA samples was continued from the step of adding 37°C for one week as set out in example 32. HCV RNA was detected using HCV Amplicor v2.0, (Roche Diagnostics, USA) and compared with HCV RNA incubated at 37°C for one week in Roche Amplicor Lysis solution before standard Amplicor v2.0 purification. Results are shown in Table 2. Stability using the method in example 32 (column entitled « MRT purification incubation at 37°C » in Table 2) was at least as good as that provided by the chaotrope guanidine in the Roche lysis buffer.

### Example 33

### Testing HCV negative plasma samples

Seven HCV negative plasma samples were tested using the method as set out in example 32 and RNA tested with HCV Amplicor v2.0, (Roche Diagnostics, USA). One sample contained added genomic DNA and another was HGV positive. All seven samples were negative for HCV but positive as expected for the internal control demonstrating the specificity of detection.

## Claims

1. A method for the stabilisation of nucleic acid from a collected biological sample, which comprises:
(a) treating the collected biological sample so that a proportion of the 2', 3' or 5'-OH positions of the nucleic acid are modified with a protecting group; and
(b) subjecting the treated sample to one or more steps to isolate nucleic acid therefrom;
wherein the modified nucleic acid is subjected to a deprotection step comprising treatment with a primary amine to remove the protecting group.

2. A method according to claim 1, wherein the biological sample comprises viruses, cells, body fluids, blood, serum or plasma.

3. A method according to claim 1 or claim 2, wherein the biological sample comprises a clinical sample or a human pathogen.

4. A method according to any of claims 1 to 3, wherein the nucleic acid is single or double stranded RNA or DNA.

5. A method according to claim 4, wherein the sample is treated with a reactant capable of covalently modifying the 2'-OH position of the ribose rings of the RNA.

6. A method according to any preceding claim, wherein step (a) is carried out in the presence of an organic solvent.

7. A method according to claim 6, wherein the organic solvent has a flashpoint above 37°C.

8. A method according to claim 6 or claim 7, wherein the organic solvent is capable of forming a homogeneous solution with human blood when mixed in a ratio of 5:1 (vol:vol).

9. A method according to any preceding claim, wherein the primary amine is ethylenediamine, diethylenetriamine, triethylenetetramine, lysine or arginine.

10. A method according to any preceding claim, wherein step (b) comprises:
(i) binding the nucleic acid to a solid phase;
(ii) optionally washing the solid phase to remove contaminants; and
(iii) optionally eluting the nucleic acid from the solid phase.

11. A method according to claim 10, wherein the solid phase comprises magnetic particles.

12. A method according to claim 10 or claim 11, wherein the solid phase contains a metal or metal ion capable of coordinating with phosphate.

13. A method according to claim 12, wherein the nucleic acid is eluted with a chelator.

14. A method according to claim 13, wherein the chelator is EGTA and elution is carried out at a pH above 9.

15. A method according to claim 13, wherein the chelator is a salt of ammonia or tetra alkylammonium.

16. A method according to claim 13, which further comprises removing the chelator from the nucleic acid by ultrafiltration, photosensitivity of the chelator or affinity purification using an affinity tag on the chelator.

17. A method according to any of claims 12 to 16, wherein the solid phase comprises hydroxylapatite.

18. A method according to claim 17, wherein the hydroxylapatite is pretreated with a phosphate-containing compound.

19. A method according to claim 17 or claim 18, wherein the hydroxylapatite is washed in step (ii) with an amine.

20. A method according to claim 19, wherein the amine is a primary amine.

21. A method according to claim 20, wherein the deprotection step comprises step (ii).

22. A method according to any of claims 9 to 18, wherein the deprotection step occurs between step (i) and step (ii).

23. A method according to claim 10 or claim 11, wherein the solid phase comprises silica.

24. A method according to claim 10 or claim 11, wherein the solid phase has immobilised thereon nucleic acid complementary to the nucleic acid targeted for isolation.

25. A method according to claim 24, wherein the nucleic acid targeted for isolation is RNA, which is subjected to the deprotection step prior to binding to the solid phase.

26. A kit for use in the method of claim 1 which comprises:
(i) a reaction system for treating the sample so that a proportion of the 2', 3' or 5'-OH positions of the nucleic acid are modified with a protecting group;
(ii) an isolation system for subjecting the treated sample to one or more steps to isolate nucleic acid therefrom; and
(iii) a primary amine for subjecting the modified nucleic acid to a deprotection step to remove the protecting group.

27. A kit according to claim 26, wherein the reaction system comprises a reactant capable of covalently modifying the 2'-OH position of the ribose rings of RNA.

28. A kit according to claim 26 or claim 27, wherein the reaction system includes an organic solvent.

29. A kit according to claim 28, wherein the organic solvent has a flashpoint above 37°C.

30. A kit according to claim 28 or claim 29, wherein the organic solvent is capable of forming a homogeneous solution with human blood when mixed in a ratio of 5:1 (vol:vol).

31. A kit according to any of claims 26 to 30, wherein the primary amine is ethylenediamine, diethylenetriamine, triethylenetetramine, lysine or arginine.

32. A kit according to any of claims 26 to 31, wherein the isolation system comprises:
(a) a solid phase for binding the nucleic acid;
(b) optionally a washing solution for washing the solid phase to remove contaminants; and
(c) optionally an elution solution for eluting the nucleic acid from the solid phase.

33. A kit according to claim 29, wherein the solid phase comprises magnetic particles.

34. A kit according to claim 32 or claim 33, wherein the solid phase contains a metal or metal ion capable of coordinating with phosphate.

35. A kit according to claim 34, wherein the elution solution comprises a chelator.

36. A kit according to claim 35, wherein the chelator is EGTA.

37. A kit according to claim 35, wherein the chelator is a salt of ammonia or tetraalkylammonium.

38. A kit according to claim 35, wherein the chelator is a photosensitive chelator or has an affinity tag.

39. A kit according to any of claims 34 to 38, wherein the solid phase comprises hydroxylapatite.

40. A kit according to claim 39, wherein the hydroxylapatite is pretreated with a phosphate-containing compound.

41. A kit according to claim 39 or claim 40, wherein the washing solution comprises an amine.

42. A kit according to claim 41, wherein the amine is the primary amine.

43. A kit according to claim 32 or claim 33, wherein the solid phase comprises silica.

44. A kit according to claim 32 or 33, wherein the solid phase has immobilised thereon nucleic acid complementary to the nucleic acid targeted for isolation.

## Patentansprüche

1. Verfahren für die Stabilisierung von Nukleinsäure aus einer gesammelten, biologischen Probe, welches umfasst:
(a) Behandeln der Probe, so dass ein Anteil der 2', 3' oder 5'-OH Positionen der Nukleinsäure mit einer Schutzgruppe modifiziert ist; und
(b) Einen oder mehrere Schritte, dem bzw. denen die behandelte Probe unterworfen wird, um hieraus Nukleinsäure zu isolieren;
wobei die modifizierte Nukleinsäure einem Entschützungsschritt unterworfen wird, der eine Behandlung mit einem primären Amin zum Entfernen der Schutzgruppe umfasst.

2. Verfahren nach Anspruch 1, wobei die biologische Probe Viren, Zellen, Körperfluide, Blut, Serum oder Plasma umfasst.

3. Verfahren nach Anspruch 1 oder Anspruch 2, wobei die biologische Probe eine klinische Probe oder ein Humanpathogen umfasst.

4. Verfahren nach einem der Ansprüche 1 bis 3, wobei die Nukleinsäure einzel- oder doppelsträngige RNA oder DNA ist.

5. Verfahren nach Anspruch 4, wobei die Probe mit einem Reaktant behandelt wird, der fähig ist die 2'-OH Position der Ribose-Ringe der RNA kovalent zu modifizieren.

6. Verfahren nach einem vorstehenden Anspruch, wobei Schritt (a) in der Gegenwart eines organischen Solvens ausgeführt wird.

7. Verfahren nach Anspruch 6, wobei das organische Solvens einen Flammpunkt (flashpoint) von über 37°C aufweist.

8. Verfahren nach Anspruch 6 oder Anspruch 7, wobei das organische Solvens zum Bilden einer homogenen Lösung mit menschlichem Blut bei Mischung in einem Verhältnis von 5:1 (vol:vol) fähig ist.

9. Verfahren nach einem vorstehenden Anspruch, wobei das primäre Amin Ethylendiamin, Diethylentriamin, Triethylentetramin, Lysin oder Arginin ist.

10. Verfahren nach einem vorstehenden Anspruch, wobei Schritt (b) umfasst :
(i) Binden der Nukleinsäure an eine feste Phase;
(ii) optional Waschen der festen Phase zum Entfernen von Kontaminantien; und
(iii) optional Eluieren der Nukleinsäure von der festen Phase.

11. Verfahren nach Anspruch 10, wobei die feste Phase magnetische Teilchen umfasst.

12. Verfahren nach Anspruch 10 oder Anspruch 11, wobei die feste Phase ein Metall oder Metallion enthält, das zum Koordinieren mit Phosphat fähig ist.

13. Verfahren nach Anspruch 12, wobei die Nukleinsäure mit einem Chelator eluiert wird.

14. Verfahren nach Anspruch 13, wobei der Chelator EGTA ist und Elution bei einem pH von über 9 ausgeführt wird.

15. Verfahren nach Anspruch 13, wobei der Chelator ein Salz von Ammoniak (ammonia) oder Tetra-alkylammonium ist.

16. Verfahren nach Anspruch 13, welches weiter umfasst:
Entfernen des Chelators von der Nukleinsäure durch Ultrafiltration, Photosensibilität des Chelators oder
Affinitätsreinigung unter Verwendung eines Affinitätstags auf dem Chelator.

17. Verfahren nach einem der Ansprüche 12 bis 16, wobei die feste Phase Hydroxylapatit umfasst.

18. Verfahren nach Anspruch 17, wobei der Hydroxylapatit mit einer Phosphat-enthaltenden Verbindung vorbehandelt wird.

19. Verfahren nach Anspruch 17 oder Anspruch 18, wobei der Hydroxylapatit in Schritt (ii) mit einem Amin gewaschen wird.

20. Verfahren nach Anspruch 19, wobei das Amin ein primäres Amin ist.

21. Verfahren nach Anspruch 20, wobei der Entschützungsschritt Schritt (ii) umfasst.

22. Verfahren nach einem der Ansprüche 9 bis 18, wobei der Entschützungsschritt zwischen Schritt (i) und Schritt (ii) erfolgt.

23. Verfahren nach Anspruch 10 oder Anspruch 11, wobei die feste Phase Siliziumoxid (Silica) umfasst.

24. Verfahren nach Anspruch 10 oder Anspruch 11, wobei die feste Phase darauf immobilisierte, zu der zu isolierenden Nukleinsäure komplementäre Nukleinsäure aufweist.

25. Verfahren nach Anspruch 24, wobei die als Ziel zur Isolierung gesetzte Nukleinsäure RNA ist, die vor einem Binden an die feste Phase dem Entschützungsschritt unterworfen wird.

26. Kit zur Verwendung in dem Verfahren nach Anspruch 1, welcher umfasst:
(i) ein Reaktionssystem zum Behandeln der Probe, so dass ein Anteil der 2', 3' oder 5'-OH Positionen der Nukleinsäure mit einer Schutzgruppe modifiziert ist;
(ii) ein Isolierungssystem um die behandelte Probe einem oder mehreren Schritten zu unterwerfen, um hieraus Nukleinsäure zu isolieren; und
(iii) ein primäres Amin, um die modifizierte Nukleinsäure einem Entschützungsschritt zum Entfernen der Schutzgruppe zu unterwerfen.

27. Kit nach Anspruch 26, worin das Reaktionssystem einen Reaktant umfasst, der fähig ist die 2'-OH Position der Ribose-Ringe von RNA kovalent zu modifizieren.

28. Kit nach Anspruch 26 oder Anspruch 27, worin das Reaktionssystem ein organisches Solvens enthält.

29. Kit nach Anspruch 28, worin das organische Solvens einen Flammpunkt (flashpoint) von über 37°C aufweist.

30. Kit nach Anspruch 28 oder Anspruch 29, worin das organische Solvens zum Bilden einer homogenen Lösung mit menschlichem Blut bei Mischung in einem Verhältnis von 5:1 (vol:vol) fähig ist.

31. Kit nach einem der Ansprüche 26 bis 30, worin das primäre Amin Ethylendiamin, Diethylentriamin, Triethylentetramin, Lysin oder Arginin ist.

32. Kit nach einem der Ansprüche 26 bis 31, worin das Isolierungssystem umfasst:
(a) eine feste Phase zum Binden der Nukleinsäure;
(b) optional eine Waschlösung zum Waschen der festen Phase zum Entfernen von Kontaminantien; und
(c) optional eine Elutionslösung zum Eluieren der Nukleinsäure von der festen Phase.

33. Kit nach Anspruch 29, worin die feste Phase magnetische Teilchen umfasst.

34. Kit nach Anspruch 32 oder Anspruch 33, worin die feste Phase ein Metall oder Metallion enthält, das zum Koordinieren mit Phosphat fähig ist.

35. Kit nach Anspruch 34, worin die Elutionslösung einen Chelator umfasst.

36. Kit nach Anspruch 35, worin der Chelator EGTA ist.

37. Kit nach Anspruch 35, worin der Chelator ein Salz von Ammoniak (ammonia) oder Tetra-alkylammonium ist.

38. Kit nach Anspruch 35, worin der Chelator ein photosensibler Chelator ist oder einen Affinitätstag aufweist.

39. Kit nach einem der Ansprüche 34 bis 38, worin die feste Phase Hydroxylapatit umfasst.

40. Kit nach Anspruch 39, worin der Hydroxylapatit mit einer Phosphat-enthaltenden Verbindung vorbehandelt ist.

41. Kit nach Anspruch 39 oder Anspruch 40, worin die Waschlösung ein Amin umfasst.

42. Kit nach Anspruch 41, worin das Amin das primäre Amin ist.

43. Kit nach Anspruch 32 oder Anspruch 33, worin die feste Phase Siliziumoxid (Silica) umfasst.

44. Kit nach Anspruch 32 oder 33, worin die feste Phase darauf immobilisierte, zu der zu isolierenden Nukleinsäure komplementäre Nukleinsäure aufweist.

## Revendications

1. Procédé pour la stabilisation d'un acide nucléique à partir d'un échantillon biologique recueilli, qui comprend les étapes consistant à :
(a) traiter l'échantillon de telle sorte qu'une proportion des positions 2'-, 3'- ou 5'-OH de l'acide nucléique soit modifiée avec un groupe protecteur ;
(b) soumettre l'échantillon traité à une ou plusieurs étapes pour isoler l'acide nucléique de celui-ci ;
dans lequel l'acide nucléique modifié est soumis à une étape de suppression de protection comprenant un traitement avec une amine primaire pour éliminer le groupe protecteur.

2. Procédé suivant la revendication 1, dans lequel l'échantillon biologique comprend des virus, des cellules, des fluides corporels, du sang, du sérum ou du plasma.

3. Procédé suivant la revendication 1 ou la revendication 2, dans lequel l'échantillon biologique comprend un échantillon clinique ou un agent pathogène humain.

4. Procédé suivant l'une quelconque des revendications 1 à 3, dans lequel l'acide nucléique est un ARN ou ADN mono- ou bicaténaire.

5. Procédé suivant la revendication 4, dans lequel l'échantillon est traité avec un corps réactionnel capable de modifier de manière covalente la position 2'-OH des noyaux ribose de l'ARN.

6. Procédé suivant l'une quelconque des revendications précédentes, dans lequel l'étape (a) est mise en oeuvre en présence d'un solvant organique.

7. Procédé suivant la revendication 6, dans lequel le solvant organique a un point d'éclair supérieur à 37°C.

8. Procédé suivant la revendication 6 ou la revendication 7, dans lequel le solvant organique est capable de former une solution homogène avec le sang humain lors du mélange en un rapport de 5:1 (en volume:volume).

9. Procédé suivant l'une quelconque des revendications précédentes, dans lequel l'amine primaire est l'éthylènediamine, la diéthylènetriamine, la triéthylènetétramine, la lysine ou l'arginine.

10. Procédé suivant l'une quelconque des revendications précédentes, dans lequel l'étape (b) comprend :
(i) la liaison de l'acide nucléique à une phase solide ;
(ii) facultativement, le lavage de la phase solide pour éliminer les contaminants ; et
(iii) facultativement, l'élution de l'acide nucléique de la phase solide.

11. Procédé suivant la revendication 10, dans lequel la phase solide comprend des particules magnétiques.

12. Procédé suivant la revendication 10 ou la revendication 11, dans lequel la phase solide contient un métal ou ion métallique apte à la coordination avec un phosphate.

13. Procédé suivant la revendication 12, dans lequel l'acide nucléique est élué avec un agent chélatant.

14. Procédé suivant la revendication 13, dans lequel l'agent chélatant est l'EGTA et l'élution est effectuée à un pH supérieur à 9.

15. Procédé suivant la revendication 13, dans lequel l'agent chélatant est un sel d'ammoniac ou de tétraalkylammonium.

16. Procédé suivant la revendication 13, qui comprend en outre l'élimination de l'agent chélatant de l'acide nucléique par ultrafiltration, photosensibilité de l'agent chélatant ou purification par affinité en utilisant un marqueur d'affinité sur l'agent chélatant.

17. Procédé suivant l'une quelconque des revendications 12 à 16, dans lequel la phase solide comprend de l'hydroxyapatite.

18. Procédé suivant la revendication 17, dans lequel l'hydroxy-apatite est prétraitée avec un composé contenant un phosphate.

19. Procédé suivant la revendication 17 ou la revendication 18, dans lequel l'hydroxy-apatite est lavée dans l'étape (ii) avec une amine.

20. Procédé suivant la revendication 19, dans lequel l'amine est une amine primaire.

21. Procédé suivant la revendication 20, dans lequel l'étape de suppression de protection comprend l'étape (ii).

22. Procédé suivant l'une quelconque des revendications 9 à 18, dans lequel l'étape de suppression de protection est mise en oeuvre entre l'étape (i) et l'étape (il).

23. Procédé suivant la revendication 10 ou la revendication 11, dans lequel la phase solide comprend de la silice.

24. Procédé suivant la revendication 10 ou la revendication 11, dans lequel la phase solide porte à l'état immobilisé un acide nucléique complémentaire de l'acide nucléique ciblé pour l'isolement.

25. Procédé suivant la revendication 24, dans lequel l'acide nucléique ciblé pour l'isolement est un ARN, qui est soumis à l'étape de suppression de protection avant liaison à la phase solide.

26. Kit pour une utilisation dans le procédé de la revendication 1, qui comprend :
(i) un système réactionnel pour le traitement de l'échantillon de telle sorte qu'une proportion des positions 2'-, 3- ou 5'-OH de l'acide nucléique soit modifiée avec un groupe protecteur ;
(ii) un système d'isolement pour soumettre l'échantillon traité à une ou plusieurs étapes pour isoler l'acide nucléique de celui-ci ; et
(iii) une amine primaire pour soumettre l'acide nucléique modifié à une étape de suppression de protection pour éliminer le groupe protecteur.

27. Kit suivant la revendication 26, dans lequel le système réactionnel comprend un corps réactionnel apte à la modification de manière covalente de la position 2'-OH des noyaux ribose de l'ARN.

28. Kit suivant la revendication 26 ou la revendication 27, dans lequel le système réactionnel comprend un solvant organique.

29. Kit suivant la revendication 28, dans lequel le solvant organique a un point d'éclair supérieur à 37°C.

30. Kit suivant la revendication 28 ou la revendication 29, dans lequel le solvant organique est capable de former une solution homogène avec le sang humain lors du mélange en un rapport de 5:1 (en volume:volume).

31. Kit suivant l'une quelconque des revendications 26 à 30, dans lequel l'amine primaire est l'éthylènediamine, la diéthylènetriamine, la triéthylènetétramine, la lysine ou l'arginine.

32. Kit suivant l'une quelconque des revendications 26 à 31, dans lequel le système d'isolement comprend :
(a) une phase solide pour la liaison de l'acide nucléique ;
(b) facultativement, une solution de lavage pour laver la phase solide pour éliminer les contaminants ; et
(c) facultativement, une solution d'élution pour éluer l'acide nucléique de la phase solide.

33. Kit suivant la revendication 29, dans lequel la phase solide comprend des particules magnétiques.

34. Kit suivant la revendication 32 ou la revendication 33, dans lequel la phase solide contient un métal ou ion métallique apte à la coordination avec un phosphate.

35. Kit suivant la revendication 34, dans lequel la solution d'élution comprend un agent chélatant.

36. Kit suivant la revendication 35, dans lequel l'agent chélatant est l'EGTA.

37. Kit suivant la revendication 35, dans lequel l'agent chélatant est un sel d'ammoniac ou de tétraalkylammonium.

38. Kit suivant la revendication 35, dans lequel l'agent chélatant est un agent chélatant photosensible ou bien possède un marqueur d'affinité.

39. Kit suivant l'une quelconque des revendications 34 à 38, dans lequel la phase solide comprend de l'hydroxyapatite.

40. Kit suivant la revendication 39, dans lequel l'hydroxy-apatite est prétraitée avec un composé contenant un phosphate.

41. Kit suivant la revendication 39 ou la revendication 40, dans lequel la solution de lavage comprend une amine.

42. Kit suivant la revendication 41, dans lequel l'amine est l'amine primaire.

43. Kit suivant la revendication 32 ou la revendication 33, dans lequel la phase solide comprend de la silice.

44. Kit suivant la revendication 32 ou 33, dans lequel la phase solide porte à l'état immobilisé un acide nucléique complémentaire de l'acide nucléique ciblé pour l'isolement.
